# EUROPEAN PATENT APPLICATION

(11) **EP 2 524 687 A2**
(43) Date of publication of application: **21.11.2012**
(21) Application number: 12003916.9
(22) Date of filing: 18.05.2012
(51) Int. Cl.: A61K 9/00, A61K 9/70, A61K 47/10, A61K 47/36, A61K 47/42

(54) **Edible jelly-form composition, jelly-form preparation and method for producing jelly-form preparation**

(30) Priority: 20.05.2011 JP 2011114043; 20.05.2011 JP 2011114044; 20.05.2011 JP 2011114046; 20.05.2011 JP 2011114048; 23.04.2012 JP 2012098245
(71) Applicant: Nitto Denko Corporation, Ibaraki-shi, Osaka 567-8680 (JP)
(72) Inventor: Asari, Daisuke, Osaka 567-8680 (JP); Shishido, Takuya, Osaka 567-8680 (JP); Hori, Mitsuhiko, Osaka 567-8680 (JP); Matsushita, Kyohei, Osaka 567-8680 (JP)
(74) Representative: Müller-Boré & Partner Patentanwälte

(57) **Abstract**

The present invention aims to provide a jelly (as a result, easy-to-swallow), intraorally soluble edible jelly composition although it is preferably free of water. The present invention relates to an edible jelly composition including: a gelling agent; and a nonvolatile organic solvent compatible with the gelling agent.

## Description

The present invention relates to an edible jelly composition gelatinized by a nonvolatile organic solvent, a jelly preparation, and a method for producing the jelly preparation.

Drugs intended for oral administration in the current market include uncoated tablets, coated tablets, capsules, powders, granules, liquids, and the like. With respect to preparations intended to be disintegrated orally and absorbed in an alimentary canal, orally disintegrating tablets and fast-dissolving oral films have already been marketed. Such a dosage form is focused on in which a drug is taken by intraorally disintegrating or dissolving the drug only with saliva and without chewing the drug because this dosage form improves benefits of patients and caregivers.
This is due to an increase in the number of patients with disability in ingestion of food and drink, in other words, those having difficulty in mastication and swallowing, involving an increase in the old people population. In addition, the Silver Science Kenkyu Houkoku (Silver Science research report) of the former Ministry of Welfare (the present Ministry of Health, Labor and Welfare) named "Koreisha ni toyosaiteki na shinkiseizai oyobi shinkihosoyoki no sakuseikenkyu" (Research of producing an optimal new preparation and packaging container for medicating elderly people), 1988, Masayasu SUGIHARA et al. reported that semisolid formulations (e.g. jelly, yogurt, and pudding) are the expected dosage form of drugs in the future.
The aforementioned backgrounds urge recent development of pharmaceutical jelly preparations, and some kinds of products have been already in the market in Japan.
All of these jelly preparations, however, are of portion-packaged type taken with a spoon or the like tools, or of pillow-packaged type taken by pushing it out from the package. Further, the jelly itself is not intraorally dissolved although it is easily dispersed by physical force upon swallowing.

Examples of water-containing jelly-like preparations disclosed so far include jelly preparations containing carrageenan, locust bean gum, and polyacrylic acid or its partially neutralized product or its salt (see Patent Document 1); pharmaceutical jelly compositions containing a jelly base and an alkaline salt (see Patent Document 2); and pharmaceutical jelly compositions containing carrageenan, guar gum, and polyacrylic acid or its partially neutralized product or its salt (Patent Document 3).
These jelly preparations, however, contain a gelling agent thermoreversible at high temperatures (about 60°C to 100°C) or contain an irreversible gelling agent which is prepared by cross-linking a gelling agent. In other words, the jelly preparations themselves are not intraorally dissolved but easily dispersed by physical force upon swallowing. Further, these conventional jelly preparations require heating at high temperatures upon preparation or contain a metal salt as a cross-linking agent. Thus, poor stability thereof may be a problem particularly in the case that the preparations contain drugs having poor heat stability or proteins or peptides strongly interacting with metal salts.

In addition, all of these conventional jelly preparations are those gelatinized.using water, and contain natural polysaccharides as a gelling agent and saccharides as an additive. Thus, fungi such as mold easily grow, so that the production process essentially includes such steps as heat sterilization and addition of an antiseptic. Furthermore, the preparations contain much water, so that they are difficult to be administered to patients who need water restriction (e.g. patients with kidney disease or heart disease). In addition, the water in the composition permeates the package to volatile, so that an expensive, highly vapor-permeable packaging material is required for good storage stability.
In order to deal with the above problems, another preparation is disclosed which is a dried, water-free film preparation and is intended to be gelatinized with saliva. Examples thereof include a preparation having a drug-containing layer and a water-swellable gel-forming layer containing a cross-linked carboxy vinyl polymer (Patent Document 4).

Such film-shaped preparations containing a water-soluble polymer, however, require a certain amount of saliva in order to be intraorally dissolved or swell, and therefore patients with dysphagia may require much time for dissolving the preparation. Further, these preparations easily absorb water, so that they easily stick to patient's oral mucosa and cause uncomfortable feeling. Particularly in the case of intraorally dissolvable film preparations, the solubility, film thickness, and size correlate with each other. As a result, they are difficult to contain a drug in an amount exceeding 100 mg. With respect to a method of producing such film-shaped preparations, one method is disclosed in which a water-soluble polymer is dissolved in water as a solvent, a drug is dissolved in this aqueous solution, and then the solution is heat-dried to produce a preparation. Particularly in the case of less heat-resistant drugs, reduction in an amount of the drug by heat is feared. In the case of liquid drugs, film-shaped preparations may be dissolved, so that a prescribed shape may not be maintained.

Examples of compositions which are re-gelatinized by adding water include dried gel formed by drying a water-containing gel containing native gellan gum (Patent Document 5) and a dried jelly product formed by dissolving at least one selected from a mixture of xanthan gum and locust bean gum and a mixture of xanthan gum and tara gum into a solvent, freeze-drying the solution to prepare a freeze-dried jelly, and then cutting the freeze-dried jelly into pieces with a size of 1 to 3 mm (Patent Document 6).
The dried gel disclosed in Patent Document 5, however, is difficult to be sufficiently re-gelatinized into a jelly composition or jelly preparation by adding water or a drug-containing solution. Further, production of the dried jelly product disclosed in Patent Document 6 needs a step of cutting a dried jelly and a step of stirring the freeze-dried jelly after adding water, so that various risks are concerned such as production losses, amount losses, and contamination with foreign matter. Therefore, the dried jelly lacks convenience as a pharmaceutical composition.

### - Patent Literature

Patent Literature 1: JP 9-187233 A
Patent Literature 2: JP 2004-99558 A
Patent Literature 3: JP 2004-99559 A
Patent Literature 4: JP 4267926 B
Patent Literature 5: JP 3671269 B
Patent Literature 6: JP 3835544 B

Under the above circumstances, the present invention aims to provide an edible jelly composition which is preferably free of water, is an easily swallowable jelly, and is intraorally dissolved, a jelly preparation containing the edible jelly composition, and a method for producing the jelly preparation.

The present inventors have performed various studies in order to solve the above problems. As a result, they have found that preparation of a gelling agent, preferably a gelling agent which is gelatinized at normal temperature to keep its solid state and is easily dissolved by water or is easily dissolved by body temperature, and gelatination of the gelling agent with a nonvolatile organic solvent that is compatible with the gelling agent enable to prepare an edible jelly composition which does not need to consider sterilization in the production, as well as vapor permeability involved in storage stability, and which is suitable for intraoral (including sublingual) and oral administration of foods and pharmaceuticals owing to its characteristics. Finally, the present inventors have completed the present invention.

That is, the present invention relates to an edible jelly composition comprising a gelling agent and a nonvolatile organic solvent compatible with the gelling agent.
The edible jelly composition of the present invention is preferably free of water.
The nonvolatile organic solvent is preferably a polyhydric alcohol having two to four OH groups and four or less carbon atoms per molecule.
The nonvolatile organic solvent is preferably at least one selected from the group consisting of glycerin, glycerin derivative, propylene glycol, and propylene glycol derivatives.
The gelling agent is preferably at least one of gelatin and a polysaccharide gelling agent.
The gelatin preferably has a random coil structure which is formed by transforming a helix structure of a slightly water soluble gelatin.
The polysaccharide gelling agent is preferably at least one selected from the group consisting of κ-carrageenan, xanthan gum, gellan gum, tamarind gum, and pectin.
The gelling agent preferably contains at least one selected from the group consisting of gelatin, κ-carrageenan, xanthan gum, deacylated gellan gum, tamarind gum, LM pectin, and HM pectin, and the nonvolatile organic solvent compatible with the gelling agent is preferably at least one of propylene glycol and a propylene glycol derivative.
The gelling agent preferably contains at least one selected from the group consisting of gelatin, κ-carrageenan, native gellan gum, deacylated gellan gum, 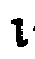 tamarind gum, LM pectin, -carrageenan/carboxymethyl cellulose sodium, 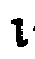 -carrageenan/sodium alginate, 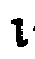 -carrageenan/xanthan gum, 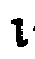 -carrageenan/λ-carrageenan, 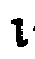 -carrageenan/deacylated gellan gum, 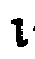 -carrageenan/psyllium seed gum, 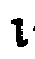 -carrageenan/powdered tragacanth, xanthan gum/deacylated gellan gum, xanthan gum/psyllium seed gum, deacylated gellan gum/carboxy vinyl polymer, deacylated gellan gum/LM pectin, deacylated gellan gum/sodium alginate, deacylated gellan gum/λ-carrageenan, deacylated gellan gum/psyllium seed gum, deacylated gellan gum/powdered tragacanth, LM pectin/psyllium seed gum, and LM pectin/powdered tragacanth, and the nonvolatile organic solvent compatible with the gelling agent is preferably at least one of glycerin and a glycerin derivative.
The nonvolatile organic solvent preferably contains at least one of glycerin and propylene glycol, and further contains, as a solution-absorption enhancer, at least one selected from the group consisting of carboxymethyl cellulose sodium, LM pectin, sodium alginate, xanthan gum, 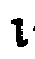 -carrageenan, λ-carrageenan, deacylated gellan gum, psyllium seed gum, and powdered tragacanth.
The amount of the gelling agent is preferably 0.1 to 40% by weight of the whole amount of the composition.
The amount of the nonvolatile organic solvent is preferably 10 to 99% by weight of the whole amount of the composition.

The present invention also relates to a jelly preparation comprising the edible jelly composition of the present invention and a drug.
The present invention also relates to a jelly preparation comprising the edible jelly composition of the present invention and a drug-containing solution.
The drug-containing solution is preferably an injectable solution or an oral solution.
The solvent of the drug-containing solution is preferably at least one selected from the group consisting of water, glycerin, and propylene glycol.

The present invention also relates to a method for producing a jelly preparation, the method comprising: mixing a gelling agent, a nonvolatile organic solvent compatible with the gelling agent, and a drug to prepare a gelling agent solution; dispensing or applying the gelling agent solution; and standing the solution to cool or cooling the solution to solidify the solution.
The present invention also relates to a method for producing a jelly preparation, the method comprising: mixing water, a gelling agent, and a nonvolatile organic solvent compatible with the gelling agent to form a mixture, and then heating the mixture to dissolve the gelling agent, to thereby prepare a nonvolatile organic solvent-containing gelling agent solution; dispensing or applying the nonvolatile organic solvent-containing gelling agent solution; standing the solution to cool or cooling the solution to solidify the solution, to thereby prepare an edible jelly composition; and adding a drug-containing solution to the edible jelly composition to prepare a jelly preparation.
The present invention also relates to a method for producing a jelly preparation, the method comprising: mixing water and a gelling agent to form a mixture, and then heating the mixture to dissolve the gelling agent, to thereby prepare a gelling agent solution; freeze-drying or spray-drying the gelling agent solution to prepare a freely soluble gelling agent; mixing the freely soluble gelling agent and a nonvolatile organic solvent compatible with the freely soluble gelling agent to form a mixture, and then heating the mixture to dissolve the freely soluble gelling agent, to thereby prepare a nonvolatile organic solvent-containing gelling agent solution; dispensing or applying the nonvolatile organic solvent-containing gelling agent solution; standing the solution to cool or cooling the solution to solidify the solution, to thereby prepare an edible jelly composition; and adding a drug-containing solution to the edible jelly composition to prepare a jelly preparation.
The present invention also relates to a method for producing a jelly preparation, the method comprising: mixing water and a gelling agent to form a mixture, and then heating the mixture to dissolve the gelling agent, to thereby prepare a gelling agent solution; freeze-drying or spray-drying the gelling agent solution to prepare a freely soluble gelling agent; mixing the freely soluble gelling agent, a nonvolatile organic solvent compatible with the freely soluble gelling agent, and a drug to prepare a nonvolatile organic solvent-containing gelling agent solution; dispensing or applying the nonvolatile organic solvent-containing gelling agent solution; and standing the solution to cool or cooling the solution to solidify the solution.
The present invention will be described in detail below.

The present invention relates to an edible jelly composition comprising a gelling agent and a nonvolatile organic solvent that is compatible with the gelling agent.
The edible jelly composition of the present invention may have any shape, and an optimum shape thereof depends on its jelly strength and its use. For example, in the case that the composition is used as a jelly preparation taken by patients or care-receivers by themselves, the edible jelly composition preferably has a strength to the extent that the preparation can sufficiently keep its shape, and is preferably a tablet, film, or sheet-form preparation. From the viewpoint of intraoral solubility, in particular, the preparation preferably has a film or sheet shape and, in this case, the thickness is preferably 30 to 5,000 µm. A product with a thickness of less than 30 µm may be poor in film strength and handleability of the product, whereas a product with a thickness of more than 5,000 µm may cause uncomfortable feeling when administered intraorally, especially sublingually.

In the case of using the edible jelly composition of the present invention as a sheet-shaped preparation, its size is not particularly limited, and the plane area is preferably from 0.5 cm² to 6.0 cm². A preparation with a plane area of smaller than 0.5 cm² may be difficult for a person to pick up and administer the preparation, whereas a preparation with a plane area of larger than 6.0 cm² may not be entirely administered intraorally, especially sublingually.
The sheet-shaped preparation may have any plane shape, and examples thereof include rectangular shapes such as a rectangle and a square, polygonal shapes such as a pentagon, a circle, an ellipse, and any other shapes. The polygonal shapes herein include not only perfect polygons but also those with slight R at its corner portions.
If a patient cannot take a preparation by oneself and a medical worker or a caregiver administers the preparation to the patient, the edible jelly composition also preferably has less strength and is cup-packed or pillow-packed so as to make it easy to administer the preparation with a spoon or the like item.

The gelling agent is a material that serves as a base of the edible jelly composition of the present invention.
Such a gelling agent is not particularly limited as long as it is edible and has a gelling property. From the viewpoint of compatibility with a nonvolatile organic solvent mentioned later, at least one of gelatin and a polysaccharide gelling agent are/is suitably used.
The term "edible" herein means that a preparation is allowed to be orally administered and is pharmaceutically acceptable.

Since the edible jelly composition of the present invention contains gelatin as the gelling agent, it is gelatinized at normal temperature and is easily dissolved intraorally by body temperature.
The gelatin is gelatinized at the lowest temperature among the thermoreversible gelling agents. Further, it enables to produce a preparation comprising the edible jelly composition of the present invention at a temperature from normal temperature to 40°C, and therefore the stability of a drug which is unstable to heat can be secured upon production.
Preferable among the gelatin is one (hereinafter, also referred to as a freely water soluble gelatin) having a random coil structure which is formed by transforming the helix structure of a slightly water soluble gelatin.
The term "freely water soluble gelatin" herein means a gelatin whose 1-g sample requires less than 1,000 mL of water at 40°C to be dissolved therein.
The molecular structure of a common gelatin is a triple helical structure, and such a gelatin is slightly soluble in water (slightly water soluble gelatin). The freely water soluble gelatin is obtainable by dissolving a slightly water soluble gelatin into water and then freeze-drying or spray-drying the solution, thereby unraveling the helical structure into a random-coiled molecular structure.
The term "slightly water soluble gelatin" herein means a gelatin whose 1-g sample requires 1,000 mL or more of water at 40°C to be dissolved therein. The term "gelatin having a random coil structure" herein means a gelatin which is obtainable by unraveling the helix structure of a slightly water soluble gelatin into a single chain state and solidifying the solution in this state; however, it may partially include a helix structure.
Even though the material of the gelatin is a slightly water soluble gelatin with a helix structure, the material mixture does not need heating upon preparing the edible jelly composition of the present invention. Thus, the composition can be stably produced. If the slightly water soluble gelatin is formed into a jelly, it shows high jelly strength. From this viewpoint, the gelatin may be prepared from a slightly water soluble gelatin which is slightly dissolved in normal-temperature water.

The gelatin preferably has a characteristic that it is not gelatinized at 32°C but is gelatinized at around 5°C when it is formed into a 10-wt% aqueous solution. Even though it is not a freely water soluble gelatin, a gelatin with such a characteristic may be of a grade that the gelatin can be sufficiently useful as the gelling agent if it has an appropriate molecular weight and hydroxyproline content therein.

The gelatin which can be used as the gelling agent is obtainable by decomposing and extracting proteins contained in skin or bone of animals using enzymes. Any gelatin obtained by acid-treating or alkali-treating proteins originated from swine, bovines, and fish can be used.
The above gelatin is preferably a fish-origin or swine-origin gelatin that can be processed at normal temperature upon production from the viewpoint of stability of a drug which is unstable to heat upon production in the case that the edible jelly composition of the present invention is used for a jelly preparation, and the viewpoint of avoiding problems such as BSE. From the above viewpoints, any gelatin may be adequate as long as it has an average molecular weight exceeding 90,000 and a hydroxyproline content of 5.2 to 9.2 mol% in its amino acid composition. Examples of such gelatin include fish-origin gelatin, and specific examples of the fish-origin gelatin include a salmon-origin gelatin (hydroxyproline content in amino acid composition: 5.4 mol%), a carp-origin gelatin (hydroxyproline content in amino acid composition: 7.6 mol%), and a tilapia-origin gelatin (hydroxyproline content in amino acid composition: 8.0 mol%). Particularly preferable is a tilapia-origin gelatin.

The amino acid composition can be determined by an analysis wherein gelatin is hydrolyzed and separated by ion-exchange chromatography, and then the amino acid composition is detected using ninhydrin.
Specific examples of the hydroxyproline content in the amino acid composition (mol%) determined by the above method are as follows..
Fowl: 10.8 mol%
Ostrich: 10.4 mol%
Mouse: 8.7 mol%
Swine: 9.4 mol%
Bovine: 9.5 mol%

Any gelatin with an average molecular weight of 50,000 to 90,000 is preferable regardless of its hydroxyproline content in the amino acid composition.
The term "average molecular weight" herein means a weight average molecular weight, and may be determined by gel filtration chromatography analysis.
Further, the term "average molecular weight" used here does not mean the molecular weight of a polypeptide chain trimer of a gelatin but means the molecular weight of each polypeptide chain monomer.

The polysaccharide gelling agent is a polysaccharide which is edible and can be gelatinized in a nonvolatile organic solvent. In other words, there are polysaccharides which are edible but are not gelatinized in a certain nonvolatile organic solvent among the polysaccharides, and such polysaccharides alone are not used as the polysaccharide gelling agent in the present invention unless they obtain a gelling property by, for example, addition of an additive or the like.
Any polysaccharide gelling agent can be used as long as it is edible and can be gelatinized. From the viewpoint of compatibility with a nonvolatile organic solvent, the polysaccharide gelling agent is preferably at least one selected from the group consisting of κ-carrageenan, xanthan gum, gellan gum, tamarind gum, and pectin. Use of at least one selected from the group consisting of κ-carrageenan, xanthan gum, gellan gum, tamarind gum, and pectin as the polysaccharide gelling agent enables to gelatinize the edible jelly composition of the present invention in a nonvolatile organic solvent and easily dissolve it into water.

The carrageenan is a straight chain sulfur-containing polysaccharide, is commonly obtainable from Chondrus crispus (red algae) by alkali extraction, and is an anionic polymer compound comprising D-galactose, 3,6-anhydro-D-galactose, and a sulfate group. Depending on the structural ratio of these components, carrageenans are classified into κ-carrageenan, 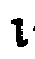 -carrageenan, and λ-carrageenan.
In the present invention, κ-carrageenan is preferable because it is gelatinized in a nonvolatile organic solvent and is gelatinized even after addition of water. 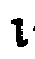 -Carrageenan alone is not gelatinized and addition of Ca²⁺ or the like is needed.
The κ-carrageenan has a gel point of around 80°C in the presence of a nonvolatile organic solvent compatible with the κ-carrageenan. Therefore, the κ-carrageenan is stable in its physical properties at around normal temperature and is excellent from the viewpoints of its use and storage.
Even in the case of adding a less heat-resistant drug (drug which is unstable to heat) to the edible jelly composition of the present invention to prepare a jelly preparation, the stability of the drug which is unstable to heat can be secured upon production by post-adding a drug-containing solution to the edible jelly composition of the present invention.
Preferable among the κ-carrageenans are those of grades with an average molecular weight of 50,000 to 500,000, and those of grades containing K⁺, Ca²⁺, and Na⁺ as counter cations. Further preferable are those of grades containing Na⁺ as a counter ion from the viewpoint of solubility.
The term "average molecular weight" used here means a weight average molecular weight, and may be determined by gel filtration chromatography analysis.

Pectins are polymer polysaccharides extracted from edible plant bodies; commonly citrus family or apples, using water. A pectin comprises galacturonic acid, and part of the carboxyl groups is methyl-esterified.
The pectins are classified into HM pectins and LM pectins depending on the ratio of the methylated galacturonic acids to all of the galacturonic acids, and those with a ratio of 50% or higher correspond to the HM pectins while those with a ratio less than 50% correspond to LM pectins. The degree of esterification (DE) of the pectin is the number of esterified carboxyl groups for 100 equivalents of the galacturonic acid. The amount of the galacturonic acid (GA) is the weight of the galacturonic acid (molecular weight: 194.1) for 100 g of a partially purified sample. These values are measured in the two steps: (1) measurement of the free acid amount by titration and (2) measurement of the free acid amount after saponification by titration.
In the edible jelly composition of the present invention, the pectin is preferable because it is gelatinized in a nonvolatile organic solvent and is also gelatinized after addition of water.
The pectin has a gel point of around 70°C in the presence of a nonvolatile organic solvent compatible with the pectin. Therefore, it has stable physical properties at around normal temperature and is excellent from the viewpoints of its use and storage.
Even in the case of adding a less heat-resistant drug (drug which is unstable to heat) to the edible jelly composition of the present invention to prepare a jelly preparation, the jelly preparation can be produced, while securing the stability of the drug which is unstable to heat upon production, by post-adding a drug-containing solution to the edible jelly composition of the present invention.
Preferable among these pectins are those of grades with an average molecular weight of 10,000 to 150,000.
The term "average molecular weight" used here means a weight average molecular weight, and may be determined by gel filtration chromatography analysis.

Gellan gum is a natural straight chain heteropolysaccharide extracellularly produced by Sphingomonas elodea, a bacterium, and it comprises repeating units of four saccharides, that is, glucose, glucuronic acid, glucose, and rhamnose.
The gellan gum is classified into native gellan gum and deacylated gellan gum depending on the presence of acetyl groups and glyceryl groups in 1,3-bonded glucose. In the edible jelly composition of the present invention, the gellan gum is preferable because it is gelatinized in a nonvolatile organic solvent and is gelatinized even after addition of water.
The gellan gum has a gel point of around 90°C in the presence of a nonvolatile organic solvent compatible with the gellan gum. Therefore, it has stable physical properties at around normal temperature, and is excellent from the viewpoints of its use and storage.
Even in the case of adding a less heat-resistant drug (drug which is unstable to heat) to the edible jelly composition of the present invention to prepare a jelly preparation, the jelly preparation can be produced, while securing the stability of the drug which is unstable to heat upon production, by post-adding a drug-containing solution to the edible jelly composition of the present invention.
Preferable among these gellan gums are those of grades with an average molecular weight of 100,000 to 700,000.
The term "average molecular weight" used here means a weight average molecular weight, and may be determined by gel filtration chromatography analysis.

The tamarind gum is a polymer polysaccharide extracted from albumens of seeds of tamarind, which is a plant of the pea family, with warm water or an alkaline aqueous solution, and its main chain comprises glucose and its side chain comprises xylose alone or xylose and galactose.
The tamarind gum has a gel point of around 80°C in the presence of a nonvolatile organic solvent compatible with the tamarind gum. Therefore, it has stable physical properties at around normal temperature, and is excellent from the viewpoints of its use and storage.
Even in the case of adding a less heat-resistant drug (drug which is unstable to heat) to the edible jelly composition of the present invention to prepare a jelly preparation, the stability of the drug which is unstable to heat can be secured upon production by post-adding a drug-containing solution to the edible jelly composition of the present invention.
Preferable among these tamarind gums are those of grades with an average molecular weight of 100,000 to 700,000.
The term "average molecular weight" used here means a weight average molecular weight, and may be determined by gel filtration chromatography analysis.

In the edible jelly composition of the present invention, the amount of the gelling agent is preferably 0.1 to 40% by weight, more preferably 0.5 to 30% by weight, and further preferably 1 to 10% by weight, of the whole amount of the edible jelly composition of the present invention. If the amount of the gelling agent is less than 0.1% by weight, the composition may not be gelatinized at normal temperature, whereas if the amount is more than 40% by weight, the composition is intraorally dissolved very slowly, likely resulting in a problem in use.

In addition to at least one of the gelatin which is suitable as the gelling agent and the polysaccharide gelling agent, the edible jelly composition of the present invention may further contain other edible polymers in combination in appropriate amounts to the extent that they do not inhibit the effects of the present invention.

Examples of the other edible polymers include: synthetic polymer compounds such as polyethylene glycol, polyvinyl alcohol, a carboxy vinyl polymer, hydroxypropyl methyl cellulose, hydroxyethyl cellulose, methyl cellulose, ethyl cellulose, hydroxypropyl cellulose with a low degree of substitution, crystalline cellulose, carboxymethyl cellulose sodium, carboxymethyl cellulose calcium, carboxymethyl cellulose, and carboxymethyl starch sodium; and polymer compounds obtainable from natural products such as dextran, casein, guar gum, tragacanth gum, acacia gum, gum arabic, psyllium seed gum, starch, zein, xanthan gum, locust bean gum, tara gum, sodium alginate, and agar. The amount of the other edible polymers is preferably 0.1 to 10% by weight of the whole amount of the edible composition of the present invention.

The edible jelly composition of the present invention may contain, as the gelling agent, two materials each of which does not serve as a gelling agent but a combination of which serves as a gelling agent. Such a combination of two materials preferably include at least one combination selected from the group consisting of
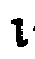 -carrageenan/carboxymethyl cellulose sodium, 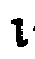 -carrageenan/sodium alginate, 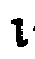 -carrageenan/xanthan gum, 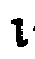 -carrageenan/λ-carrageenan, 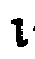 -carrageenan/deacylated gellan gum, 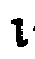 -carrageenan/psyllium seed gum, 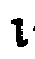 -carrageenan/powdered tragacanth, xanthan gum/deacylated gellan gum, xanthan gum/psyllium seed gum, deacylated gellan gum/carboxy vinyl polymer, deacylated gellan gum/LM pectin, deacylated gellan gum/sodium alginate, deacylated gellan gum/λ-carrageenan, deacylated gellan gum/psyllium seed gum, deacylated gellan gum/powdered tragacanth, LM pectin/psyllium seed gum, and LM pectin/powdered tragacanth.

In the edible jelly composition of the present invention, the gelling agent preferably contains at least one selected from the group consisting of gelatin, κ-carrageenan, tamarind gum, deacylated gellan gum, LM pectin, and HM pectin, and the later-mentioned nonvolatile organic solvent is preferably at least one of propylene glycol and a propylene glycol derivative. Combination of such a gelling agent and a nonvolatile organic solvent enables to prepare a water-free edible jelly composition, and this jelly composition can absorb a drug-containing solution, which is to be post-added, to provide a jelly preparation.

Further, in the edible jelly composition of the present invention, the gelling agent preferably contains at least one selected from the group consisting of gelatin, κ-carrageenan, native gellan gum, deacylated gellan gum, tamarind gum, LM pectin, 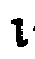 -carrageenan/carboxymethyl cellulose sodium, 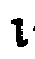 -carrageenan/sodium alginate, 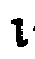 -carrageenan/xanthan gum, 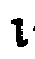 -carrageenan/λ-carrageenan, 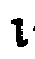 -carrageenan/deacylated gellan gum, 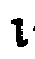 -carrageenan/psyllium seed gum, 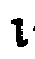 -carrageenan/powdered tragacanth, xanthan gum/deacylated gellan gum, xanthan gum/psyllium seed gum, deacylated gellan gum/carboxy vinyl polymer, deacylated gellan gum/LM pectin, deacylated gellan gum/sodium alginate, deacylated gellan gum/λ-carrageenan, deacylated gellan gum/psyllium seed gum, deacylated gellan gum/powdered tragacanth, LM pectin/psyllium seed gum, and LM pectin/powdered tragacanth, and the later-mentioned nonvolatile organic solvent is preferably at least one of glycerin and a glycerin derivative. Combination of such a gelling agent and a nonvolatile organic solvent enables to prepare a water-free edible jelly composition, and this jelly composition can absorb a drug-containing solution, which is to be post-added, to provide a jelly preparation.

Furthermore, in the edible jelly composition of the present invention, the gelling agent preferably contains at least one selected from the group consisting of gelatin, κ-carrageenan, tamarind gum, and HM pectin, and the later-mentioned nonvolatile organic solvent is preferably at least one of propylene glycol and a propylene glycol derivative. Combination of such a gelling agent and a nonvolatile organic solvent enables to prepare a water-free edible jelly composition, and this jelly composition can absorb a drug-containing solution, which is to be post-added, to provide a jelly preparation.

The nonvolatile organic solvent that is compatible with the gelling agent helps dissolution of the edible jelly composition of the present invention. The dissolution time of the edible jelly composition of the present invention can be easily adjusted by adjusting the amount of the nonvolatile organic solvent in the edible jelly composition of the present invention. Therefore, the edible jelly composition of the present invention is suitable for both of a case that the composition is dissolved intraorally to be administered and a case that the composition is slowly dissolved intraorally, especially sublingually, to sustain-release a drug.
The amount of the nonvolatile organic solvent is preferably 10 to 99% by weight, and more preferably 20 to 80% by weight, of the whole amount of the edible jelly composition of the present invention. If the amount thereof is less than 10% by weight, intraoral solubility is extremely poor, likely resulting in a problem in use. If the amount is more than 99% by weight, storage stability in terms of physical properties such as shape retentivity at normal temperature may be poor.

Any nonvolatile organic solvent may be used which is compatible with the gelling agent and is edible. Suitably used are polyhydric alcohols having two to four OH groups and four or less carbon atoms per molecule. A polyhydric alcohol having less than two OH groups per molecule may be less compatible with the gelling agent such as gelatin. A polyhydric alcohol having more than four OH groups per molecule may have a higher melting point, so that it is in a solid state at normal temperature and is difficult to be used as a solvent.
A polyhydric alcohol having more than four carbon atoms may be less compatible with the gelling agent such as gelatin.

Suitable as such a polyhydric alcohol is at least one selected from the group consisting of glycerin, propylene glycol, and ethylene glycol.
Each of these nonvolatile organic solvents may be used alone, or two or more of these may be used in combination. Particularly in the case that the edible jelly composition of the present invention contains a large amount of the solvents, the polyhydric alcohol is preferably at least one of glycerin and propylene glycol from the viewpoint of safety in administration to humans.
In the case that the edible jelly composition of the present invention contains at least one of glycerin and propylene glycol as the nonvolatile organic solvent, it preferably further contains, as a solution-absorption enhancer, at least one selected from the group consisting of carboxymethyl cellulose sodium, LM pectin, sodium alginate, xanthan gum, 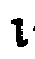 -carrageenan, λ-carrageenan, deacylated gellan gum, psyllium seed gum, and powdered tragacanth for the purpose of increasing the speed of absorbing the nonvolatile organic solvent.

The edible jelly composition of the present invention is preferably free of water.
Since the edible jelly composition of the present invention is free of water, a sterilization step or addition of an antiseptic is not needed in the production process, which are required for production of common edible jelly compositions. Therefore, the present invention is advantageous in the production cost. Further, the composition of the present invention is suitably applied to forms of dietary supplements and pharmaceutical products for patients who need water restriction.
The phrase "free of water" herein includes the case that the composition contains substantially no water. The phrase means, for example, that the amount of water is 5% by weight or less, preferably 2.5% by weight or less, and more preferably 1% by weight or less, of the whole amount of the edible jelly composition of the present invention.

The edible jelly composition of the present invention may contain an antifoaming agent.
Examples of the antifoaming agent include sorbitan fatty acid esters and sucrose fatty acid esters.
Examples of the sorbitan fatty acid esters include sorbitan monooleate, sorbitan trioleate, sorbitan sesquioleate, sorbitan cocoate, and polyoxyethylene sorbitan fatty acid esters.
Examples of the sucrose fatty acid esters include sucrose stearate, sucrose oleate, sucrose palmitate, sucrose myristate, sucrose behenate, sucrose erucate, and sucrose-mixed fatty acid esters.

In addition to the aforementioned substances, the edible jelly composition of the present invention may appropriately contain other components such as perfumes, flavoring agents, sweetening agents, coloring agents, antiseptics, antioxidants, stabilizing agents, and surfactants, if necessary, as components constituting the base of the edible jelly composition.

The edible jelly composition of the present invention may contain an additive which improves the physical properties and solubility.
Examples of the additive include the following monosaccharides, disaccharides, tri- to hexa-saccharides, and alcohols of these saccharides.
Examples of the monosaccharides include: aldotetroses such as erythrose and threose; aldopentoses such as ribose, lyxose, xylose, and arabinose; aldohexoses such as allose, talose, gulose, glucose, altrose, mannose, galactose, and idose; ketotetroses such as erythrulose; ketopentoses such as xylulose and ribulose; and ketohexoses such as psicose, fructose, sorbose, and tagatose. Examples of the disaccharides include: α-diglucosides such as trehalose, kojibiose, nigerose, maltose, and isomaltose; β-diglucosides such as isotrehalose, sophorose, laminaribiose, cellobiose, and genthiobiose; and α,β-diglucosides such as neotrehalose, as well as lactose, sucrose, and isomaltulose (palatinose). Examples of the trisaccharides include raffinose. Examples of the tri- to hexa-oligosaccharides include fructooligosaccharide, galactooligosaccharide, xylooligosaccharide, isomaltooligosaccharide, chitin oligosaccharide, chitosan oligosaccharide, oligoglucosamine, dextrin, and cyclic oligosaccharides such as cyclodextrin.

Examples of alcohols of the monosaccharides include tetritols such as erythritol, D-threitol, and L-threitol; pentitols such as D-arabinitol and xylitol; hexitols such as D-iditol, galactitol (dulcitol), D-glucitol (sorbitol), and mannitol; and cyclitols such as inositol. Examples of alcohols of the disaccharides include maltitol, lactitol, and reduced palatinose (isomalt). Examples of alcohols of the oligosaccharides include pentaerythritol and reduced maltose syrup.
In the edible jelly composition of the present invention, the saccharides or alcohols of the saccharides may be optionally substituted, and each of these may be used alone or two or more of these may be used in admixture.

The saccharides or alcohols of the saccharides are preferably mono- to tri-saccharides or alcohols of these saccharides in order to easily dissolve a sheet-shaped preparation comprising the edible jelly composition of the present invention intraorally and to prevent a great change in viscosity of the solution in the production process.

The amount of the additive is preferably 1 to 80% by weight, and more preferably 5 to 70% by weight, of the whole amount of the edible jelly composition of the present invention. If the amount is less than 1% by weight, sufficient physical properties may not be secured in its use. If the amount is more than 80% by weight, it may be difficult to control the physical properties of a sheet- or film-shaped preparation which is prepared from the edible jelly composition of the present invention using the additive.

A jelly preparation may be provided by adding a drug to the edible jelly composition of the present invention.
Further, a jelly preparation may be provided by post-adding a drug-containing solution to the edible jelly composition of the present invention.
The term "drug" herein means a substance which has any biological activity. Such a jelly preparation comprising the edible jelly composition of the present invention and a drug, and such a jelly preparation obtainable by post-adding a drug-containing solution to the edible jelly composition of the present invention each are one aspect of the present invention.

The drug in the jelly preparation of the present invention is preferably a drug which can be administered to mammals, such as human, sublingually, intraorally, or intraintestinally, that is, a drug which can be orally administered. Specific examples of such a drug include general anesthetics, sedative hypnotics, antiepileptic drugs, antipyretic-analgesic-antiinflammatory drugs, anti-vertiginous drugs, psychoneurotic drugs, central-nervous-system drugs, antidementia drugs, local anesthetics, skeletal muscle relaxants, autonomic-nervous-system drugs, antispasmodics, antiparkinson drugs, antihistamines, cardiotonics, antiarrhythmic drugs, diuretics, hypotensive agents, vasoconstrictors, coronary vasodilators, peripheral vasodilators, antiarteriosclerotic drugs, circulatory-system drugs, respiratory stimulants, cough suppressants and expectorants, hormone drugs, external preparations for purulent diseases, analgesic-antipruritic-astringent-antiphlogistic drugs, drugs for parasitic skin diseases, hemostatics, gout remedies, antidiabetic drugs, antineoplastics, antibiotics, chemotherapeutic drugs, narcotic drugs, smoking cessation aids, allergens for hyposensitization therapy, and vaccines.

The drug can exist in a pressure-sensitive agent layer in an amount enough to give a desired result in treatment of diseases, conditions, or disorders, for example, a desired treatment result; such an amount is called an effective amount herein.
The term "drug in an effective amount" herein means, for example, a drug in an amount which causes no toxicity but is enough to give a selected effect for a predetermined period. Such an amount can be easily determined by the skilled person.

The drug may be a solid drug or may be a liquid drug. The term "solid drug" herein means a drug which is in a solid state at room temperature (25°C), that is, a drug having a melting point higher than 25°C. The term "melting point" herein means a value measured using a DSC, model DSC 6220 (Seiko Instruments Inc. (SII)).
The term "liquid drug" herein means a drug having fluidity at room temperature (25°C), that is, a drug having a viscosity of 0.05 to 100,000 mPa·s at room temperature (25°C). The viscosity of the drug may be measured using an E-type viscometer while the temperature of the drug is kept at 25°C.
The drug-containing solution that contains a drug is preferably an injectable solution or an oral solution.

The concentration of the drug depends on its properties, and is generally 1×10⁻¹⁰ to 80% by weight of the whole amount of the jelly preparation of the present invention. If the drug concentration is lower than 1×10⁻¹⁰% by weight, many drugs may not show their efficacy in terms of the clinical effects. If the drug concentration is higher than 80% by weight, the physical properties of the edible jelly composition may be extremely deteriorated and the edible jelly composition may have a problem in its shape retentivity.

The solvent in the drug-containing solution is preferably at least one selected from the group consisting of water, glycerin, and propylene glycol from the viewpoint of the safety of administration to humans.

In the jelly preparation of the present invention, the amount of the gelling agent is preferably 0.1 to 40% by weight, more preferably 0.5 to 30% by weight, and further preferably 1 to 20% by weight, of the whole amount of the jelly preparation. If the amount thereof is less than 0.1% by weight, the preparation may not be gelatinized at normal temperature. If the amount is more than 40% by weight, the intraoral solubility of the preparation may be very poor, likely resulting in a problem in its use.

In the jelly preparation of the present invention, the aforementioned edible jelly composition of the present invention preferably contains a nonvolatile organic solvent compatible with the gelling agent. The amount of the nonvolatile organic solvent is preferably 10 to 99% by weight, and more preferably 20 to 80% by weight, of the whole amount of the jelly preparation. If the amount of the nonvolatile organic solvent is less than 10% by weight, the intraoral solubility of the preparation may be very poor, likely resulting in a problem in its use. If the amount is more than 99% by weight, the storage stability of the preparation in its physical properties, such as the shape retentivity at normal temperature, may be deteriorated.

In the jelly preparation of the present invention, preferably, the gelling agent and the nonvolatile organic solvent are appropriately selected and used in combination so as to exert the effects of the present invention.
With respect to the combination of the gelling agent and the nonvolatile organic solvent in the present invention, the gelling agent is preferably at least one selected from the group consisting of gelatin, κ-carrageenan, native gellan gum, deacylated gellan gum, tamarind gum, LM pectin, 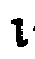 -carrageenan/carboxymethyl cellulose sodium, 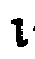 -carrageenan/sodium alginate, 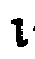 -carrageenan/xanthan gum, 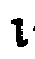 -carrageenan/λ-carrageenan, 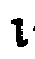 -carrageenan/deacylated gellan gum, 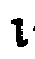 -carrageenan/psyllium seed gum, 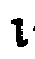 -carrageenan/powdered tragacanth, xanthan gum/deacylated gellan gum, xanthan gum/psyllium seed gum, deacylated gellan gum/carboxy vinyl polymer, deacylated gellan gum/LM pectin, deacylated gellan gum/sodium alginate, deacylated gellan gum/λ-carrageenan, deacylated gellan gum/psyllium seed gum, deacylated gellan gum/powdered tragacanth, LM pectin/psyllium seed gum, and LM pectin/powdered tragacanth, whereas the nonvolatile organic solvent that is compatible with the gelling agent is preferably at least one of glycerin and a glycerin derivative.
With respect to other combinations, the gelling agent is preferably at least one selected from the group consisting of gelatin, κ-carrageenan, xanthan gum, deacylated gellan gum, tamarind gum, LM pectin, and HM pectin, whereas the nonvolatile organic solvent compatible with the gelling agent is preferably at least one of propylene glycol and a propylene glycol derivative.

The jelly preparation of the present invention can be produced, for example, by a method comprising: mixing a gelling agent, a nonvolatile organic solvent compatible with the gelling agent, and a drug to prepare a gelling agent solution; dispensing or applying the gelling agent solution; and standing the solution to cool or cooling the solution to solidify the solution.
Such a method for producing the jelly preparation of the present invention is also one aspect of the present invention (hereinafter, also referred to as a first method for producing the jelly preparation of the present invention).
If no drug is added in the first method for producing the jelly preparation of the present invention, the jelly composition of the present invention can be produced.

In the step of preparing the gelling agent solution, the gelling agent and the other additives are first dissolved into a predetermined amount of the nonvolatile organic solvent at normal temperature or under heating, and the additives which are not soluble to the nonvolatile organic solvent are uniformly dispersed therein.
In the case that the gelling agent is the aforementioned freely water soluble gelatin, the gelling agent solution is prepared by the above method. If the gelling agent is a slightly water soluble gelatin, the slightly water soluble gelatin is not easily dissolved into a nonvolatile organic solvent, in general. Therefore, the gelling agent solution may be prepared, for example, as follows: the slightly water soluble gelatin is dissolved into water under heating to prepare a gelatin solution; the solution is freeze-dried or spray-dried to prepare a gelatin having a random coil structure whose solubility to the nonvolatile organic solvent is increased; and then the gelatin is mixed with the nonvolatile organic solvent. Alternatively, the gelling agent solution may be prepared as follows: the slightly water soluble gelatin is dissolved into water under heating to prepare a solution; and then a predetermined amount of the solution is added to the nonvolatile organic solvent. Such a process enables to dissolve even a slightly water soluble gelatin into a nonvolatile organic solvent.
If the drug is stable to heat, it is added to the solvent together with the components such as the gelling agent to prepare a gelling agent solution. If the drug is unstable to heat, the components such as the gelling agent are dissolved into the solvent, the solution is cooled down to from normal temperature to around 35°C, the drug unstable to heat is added thereto, and then the solution and the drug are stir-mixed to prepare a gelling agent solution. The drug may be added in the later-mentioned step of dispensing or applying the gelling agent solution.
If bubbles are generated upon preparing the gelling agent solution, the solution is left to stand for a long time or deaerated under vacuum or under reduced pressure to sufficiently remove the bubbles.

The freeze-drying of the gelatin solution may be performed by, for example, a method in which the gelatin solution is frozen using liquid nitrogen, and then subjected to a treatment using a known freeze-drier.
The spray-drying of the gelatin solution may be performed by, for example, a method in which the solution is sprayed using a spray drier, and then the sprayed solution is dried at around 30°C. If water remains in the obtained gelatin particles, the particles may be subjected to secondary drying or reduced-pressure drying at around 20°C to 30°C.

In the step of dispensing or applying the gelling agent solution and the step of standing the solution to cool or cooling the solution to solidify the solution, the gelling agent solution is dispensed in portions (each in a predetermined amount) into plastic or aluminum blister cases or pillow packs each having a desired size at 28°C to 32°C, and immediately after dispensing the solution, each portion of the solution is cooled and solidified. Instead of the dispensing step, an appropriate amount of the gelling agent solution is applied to a plastic release film, the applied solution is cooled and solidified, and then the solidified product is cut into pieces each having a desired size.

If the gelling agent solution contains water, the solution needs to be dried under heating or dried under reduced pressure before it is cooled to be solidified in the present step.
In order to adjust the amount of the nonvolatile organic solvent contained in a jelly preparation to be produced, the solution may be subjected to cool-air drying or a cold vacuum drying after the present step.

Further, the jelly preparation of the present invention can be produced by a method comprising: mixing water, a gelling agent, and a nonvolatile organic solvent compatible with the gelling agent to form a mixture, and then heating the mixture to dissolve the gelling agent, to thereby preparing a nonvolatile organic solvent-containing gelling agent solution; dispensing or applying the nonvolatile organic solvent-containing gelling agent solution; standing the solution to cool or cooling the solution to solidify the solution, to thereby prepare an edible jelly composition; and adding a drug-containing solution to the edible jelly composition to prepare a jelly preparation.
Such a method for producing the jelly preparation of the present invention is also one aspect of the present invention (hereinafter, also referred to as a second method for producing the jelly preparation of the present invention).
If the steps from the start to preparation of the edible jelly composition in the second method for producing the jelly preparation of the present invention are performed, the edible jelly composition of the present invention can be produced.

In the step of preparing the nonvolatile organic solvent-containing gelling agent solution, the aforementioned gelling agent such as at least one of gelatin and polysaccharide gelling agent and other additives are dissolved into a predetermined amount of the nonvolatile organic solvent at normal temperature or under heating, and additives which are not soluble to the nonvolatile organic solvent are uniformly dispersed.
If bubbles are generated upon preparation of the nonvolatile organic solvent-containing gelling agent solution, the solution is left standing for a long time or deaerated under vacuum or under reduced pressure to sufficiently remove the bubbles.

Further, the step of preparing the nonvolatile organic solvent-containing gelling solution may be as follows: in advance, water and a gelling agent are mixed to form a mixture, and the mixture is heated to dissolve the gelling agent, to thereby prepare a gelling agent solution, and next the gelling agent solution is freeze-dried or spray-dried to prepare a freely soluble gelling agent; and the freely soluble gelling agent and a nonvolatile organic solvent compatible with the freely soluble gelling agent are mixed to form a mixture, and the mixture is heated to dissolve the freely soluble gelling agent, to thereby prepare a nonvolatile organic solvent-containing gelling agent solution. Such a step enables to dissolve the gelling agent into the nonvolatile organic solvent, even if the gelling agent is slightly soluble to the nonvolatile organic solvent. Such a method for producing a jelly preparation in which the step of preparing a gelling agent solution and the step of preparing a freely soluble gelling agent are performed before the step of preparing a nonvolatile organic solvent-containing gelling solution is also one aspect of the present invention (hereinafter, also referred to as a third method for producing the jelly preparation of the present invention).

The freeze-drying of the gelling agent solution may be performed by, for example, a method in which the gelling agent solution is frozen using liquid nitrogen, and then subjected to a treatment using a known freeze-drier.
The spray-drying of the gelling agent solution may be performed by, for example, a method in which the solution is sprayed using a spray drier, and then the sprayed solution is dried at around 30°C. If water remains in the obtained gelling agent particles, the particles may be subjected to secondary drying or reduced-pressure drying at around 20°C to 30°C.

In the step of dispensing or applying the nonvolatile organic solvent-containing gelling agent solution and the step of standing the solution to cool or cooling the solution to thereby solidify the solution in the second and third methods for producing the jelly preparation of the present invention, the nonvolatile organic solvent-containing gelling agent solution are dispensed in portions (each in a predetermined amount) into plastic or aluminum blister cases or pillow packs each having a desired size at 40°C to 80°C, and immediately after dispensing the solution, each portion of the solution is cooled and solidified. Instead of the dispensing step, an appropriate amount of the nonvolatile organic solvent-containing gelling agent solution is applied to a plastic release film, the applied solution is cooled and solidified, and then the solidified product is cut into pieces each having a desired size.

In order to remove water in the nonvolatile organic solvent-containing gelling agent solution, the solution needs to be dried under heating or dried under reduced pressure before it is cooled to be solidified in the present step.
In order to adjust the amount of the nonvolatile organic solvent contained in a jelly preparation to be produced, the solution may be subjected to cool-air drying or a cold vacuum drying after the present step.

In the step of preparing a jelly preparation, the aforementioned drug-containing solution is applied to the edible jelly composition, and thereby the drug-containing solution is allowed to permeate into the edible jelly composition.
As mentioned above, the drug-containing solution is added after the edible jelly composition is formed. Thus, even a drug which is unstable to heat can be suitably used in the second and third methods for producing the jelly preparation of the present invention.

Furthermore, the jelly preparation of the present invention can be produced by a method comprising: mixing water and a gelling agent to form a mixture, and then heating the mixture to dissolve the gelling agent, to thereby prepare a gelling agent solution; freeze-drying or spray-drying the gelling agent solution to prepare a freely soluble gelling agent; mixing the freely soluble gelling agent, a nonvolatile organic solvent compatible with the freely soluble gelling agent, and a drug to prepare a nonvolatile organic solvent-containing gelling agent solution; dispensing or applying the nonvolatile organic solvent-containing gelling agent solution; and standing the solution to cool or cooling the solution to solidify the solution.
Such a method for producing the jelly preparation of the present invention is also one aspect of the present invention (hereinafter, also referred to as a fourth method for producing the jelly preparation of the present invention).
If no drug is added in the fourth method for producing the jelly preparation of the present invention, the jelly composition of the present invention can be produced.

The step of preparing a gelling agent solution and the step of preparing a freely soluble gelling agent are the same as the step of preparing a gelling agent solution and the step of preparing a freely soluble gelling agent in the third method for producing the jelly preparation of the present invention, respectively. Such steps enable to dissolve the gelling agent into the nonvolatile organic solvent even in the case of the gelling agent that is slightly soluble to the nonvolatile organic solvent.

In the step of preparing a nonvolatile organic solvent-containing gelling agent solution in the fourth method for producing the jelly preparation of the present invention, the freely soluble gelling agent obtained in the above step and other additives are first dissolved in a predetermined amount of the nonvolatile organic solvent at normal temperature or under heating, and additives which are not soluble to the nonvolatile organic solvent are uniformly dispersed.
The freely soluble gelling agent can be dissolved in the nonvolatile organic solvent even in the case that the gelling agent as a material of the freely soluble gelling agent is not easily dissolved into the nonvolatile organic solvent. Therefore, both in the case of a drug which is stable to heat and in the case of a drug which is unstable to heat, the nonvolatile organic solvent-containing gelling agent solution can be prepared by appropriate stir-mixing.
Further, the drug may be added in the step of dispensing or applying the nonvolatile organic solvent-containing gelling agent solution.
If bubbles are generated upon preparing the gelling agent solution, the solution is left standing for a long time or deaerated under vacuum or under reduced pressure to sufficiently remove the bubbles.

In the step of dispensing or applying the nonvolatile organic solvent-containing gelling agent solution and the step of standing the solution to cool or cooling the solution to thereby solidify the solution, the nonvolatile organic solvent-containing gelling agent solution are dispensed in portions (each in a predetermined amount) into plastic or aluminum blister cases or pillow packs each having a desired size at 40°C to 80°C, and immediately after dispensing the solution, each portion of the solution is cooled and solidified. Instead of the dispensing step, an appropriate amount of the nonvolatile organic solvent-containing gelling agent solution is applied to a plastic release film, the applied solution is cooled and solidified, and then the solidified product is cut into pieces each having a desired size.

If the nonvolatile organic solvent-containing gelling agent solution contains water, the solution needs to be dried under heating or dried under reduced pressure before it is cooled to be solidified in the present step.
In order to adjust the amount of the nonvolatile organic solvent contained in a jelly preparation to be produced, the solution may be subjected to cool-air drying or cold vacuum drying after the present step.

Preferably, the sheet- or film-shaped jelly preparations obtained by the above methods are airtightly packed and thereby prepared as products, if necessary.
The method for producing a jelly preparation of the present invention is very useful in that a composition can be prepared at temperatures as low as 35°C or lower, preferably 30°C or lower, especially in the case of using a drug which is unstable to heat.

The edible jelly composition of the present invention is a jelly-like (as a result, easy-to-swallow), intraorally soluble edible jelly composition although it is a jelly-like composition preferably free of water.
Further, the dissolution time of the edible jelly composition of the present invention can be easily adjusted by adjusting the amount of the nonvolatile organic solvent. Since the dissolution time is adjustable, the composition of the present invention can be suitably formed into a dosage form of a pharmaceutical product to be absorbed through the oral mucosa and sublingual mucosa that requires intraoral residence time, and is also suitable for sublingual hyposensitization therapy in which the body is sensitized to allergens through the sublingual mucosa.
Since the edible jelly composition of the present invention is free of water, the composition does not need a sterilization step or addition of an antiseptic, which are required for common jelly. Thus, the composition of the present invention is advantageous in the production cost, and is suitable for forms of dietary supplements and pharmaceutical products for patients who need water restriction.
In addition, the edible jelly composition of the present invention can be easily solidified by post-adding a drug-containing solution such as injections and oral solutions, and thereby a jelly preparation is prepared. The edible jelly composition is very suitable for solidifying not only proteins and peptides, which are very unstable to heat, but also tailored drugs prepared by high-mix low-volume production.
The edible jelly composition of the present invention suitably contains gelatin and polyols such as glycerin and propylene glycol which are commonly known to improve storage stability of proteins and peptides. Thus, the composition is expected to stably maintain, in particular, proteins and peptides.
The edible jelly composition of the present invention may naturally be swallowed as it is, or may be rapidly dissolved intraorally and then swallowed. In addition, the intraoral dissolution time is adjustable, and thus the composition can be expected to be absorbed through the oral mucosa and sublingual mucosa. Since the composition can be perfectly dissolved by body temperature and thus causes no feeling of residues, and since the composition is free of water, the jelly preparation comprising the edible jelly composition of the present invention can greatly improve the QOL of patients who need water restriction, patients with dysphagia, and caregivers.

The following description is given to illustrate the present invention by way of examples, but the present invention is not limited to these examples.

### (Example 1)

Fish-derived freely water soluble gelatin (0.5 parts by weight, water-soluble gelatin CSF, from Nippi Inc.) was added to propylene glycol (9.5 parts by weight, from Wako Pure Chemical Industries, Ltd.) and dissolved therein while agitating at a temperature of 60°C. After the dissolution, a 1-g portion of the solution was dispensed to a 5-cm² plastic blister case (Cryomold (square type) No. 3, from Sakura Finetek Japan Co., Ltd.), and cooled at 2°C to 8°C overnight. In this manner, an edible composition was provided.

### (Examples 2 to 7)

Edible compositions were prepared respectively using the materials shown in Table 1 in the same manner as in

### Example 1.

In Examples 5 to 7, glycerin was used instead of the propylene glycol (from Wako Pure Chemical Industries, Ltd.). In Examples 6 and 7, freely water-soluble swine-derived gelatin (water soluble gelatin CS, from Nippi Inc.) was used instead of the freely water soluble fish-derived gelatin (water soluble gelatin CSF, from Nippi Inc.).

### (Comparative Example 1)

Fish-derived freely water soluble gelatin (1.0 part by weight, water soluble gelatin CSF, from Nippi Inc.) was added to polyethylene glycol 400 (9.0 parts by weight, from Wako Pure Chemical Industries, Ltd.) and agitated at a temperature of 60°C. The gelatin was not dissolved. Then, the mixture was heated up to 80°C and agitated, but the gelatin was not dissolved. A 1-g portion of the mixture in which the precipitate remained was dispensed as it was to a 5-cm² plastic blister case (Cryomold (square type) No. 3, from Sakura Finetek Japan Co., Ltd.), and cooled at 2°C to 8°C overnight. In this manner, an edible composition was provided.

### (Comparative Example 2)

Swine-derived freely water soluble gelatin (1.0 part by weight, water soluble gelatin CS, from Nippi Inc.) was added to polyethylene glycol 400 (9.0 parts by weight, from Wako Pure Chemical Industries, Ltd.) and agitated at a temperature of 60°C. The gelatin was not dissolved. Then, the mixture was heated up to 80°C and agitated but the gelatin was not dissolved. A 1-g portion of the mixture in which the precipitate remained was dispensed as it was to a 5-cm² plastic blister case (Cryomold (square type) No. 3, from Sakura Finetek Japan Co., Ltd.), and cooled at 2°C to 8°C overnight. In this manner, an edible composition was provided.

**[Table 1]**

| Ingredient | Example [parts by weight] | | | | | | | Comparative Example [parts by weight] | |
|---|---|---|---|---|---|---|---|---|---|
| | 1 | 2 | 3 | 4 | 5 | 6 | 7 | 1 | 2 |
| Fish-derived water-soluble gelatin | 0.5 | 1.0 | 1.5 | 2.0 | 1.0 | - | - | 1.0 | - |
| Swine-derived water-soluble gelatin | - | - | - | - | - | 1.0 | 1.0 | - | 1.0 |
| Propylene glycol | 9.5 | 9.0 | 8.5 | 8.0 | - | 9.0 | - | - | - |
| Glycerin | - | - | - | - | 9.0 | - | 9.0 | - | - |
| PEG400 | - | - | - | - | - | - | - | 9.0 | 9.0 |
| Dispensed amount [g/blister] | 1.0 | 1.0 | 1.0 | 1.0 | 1.0 | 1.0 | 1.0 | 1.0 | 1.0 |
| Size [cm²] | 5 | 5 | 5 | 5 | 5 | 5 | 5 | 5 | 5 |

### (Example 8)

Swine-derived freely water soluble gelatin (5 parts by weight, water soluble gelatin CS, from Nippi Inc.) was added to glycerin (43.9 parts by weight, from Wako Pure Chemical Industries, Ltd.) and heated and dissolved at a temperature of 60°C. To the solution was added precipitated calcium carbonate (50 parts by weight, from Bihoku Funka Kogyo Co., Ltd.) as a drug, sucralose (0.1 parts by weight, from San-Ei Gen F.F.I., Inc.), and citric acid (1.0 part by weight, citric acid hydrate (Japanese Pharmacopoeia) from Komatsuya Corporation) and agitated at a temperature of 50°C. Thereafter, a 2.0-g portion of the solution was dispensed to a 5-cm² plastic blister case (Cryomold (square type) No. 3, from Sakura Finetek Japan Co., Ltd.), and cooled at 2°C to 8°C overnight. In this manner, a film preparation was provided.

### (Examples 9 and 10)

Film preparations were prepared respectively using the materials shown in Table 2 in the same manner as in Example 8. Instead of the precipitated calcium carbonate (from Bihoku Funka Kogyo Co., Ltd.) as the drug, lanthanum carbonate hydrate (from Wako Pure Chemical Industries, Ltd.) was used in Example 9, and ethyl icosapentate (from Wako Pure Chemical Industries, Ltd.) was used in Example 10.

**[Table 2]**

| Ingredient | Example [parts by weight] | | |
|---|---|---|---|
| | 8 | 9 | 10 |
| Precipitated calcium carbonate | 50.0 | - | - |
| Lanthanum carbonate hydrate | - | 40.0 | - |
| Ethyl icosapentate | - | - | 30.0 |
| Swine-derived water-soluble gelatin | 5.0 | 6.0 | 7.0 |
| Sucralose | 0.1 | 0.1 | 0.1 |
| Citric acid | 1.0 | 1.0 | 1.0 |
| Glycerin | 43.9 | 52.9 | 60.9 |
| Dispensed amount [g/blister] | 2.0 | 2.0 | 2.0 |
| Size [cm²] | 5 | 5 | 5 |

### (Example 11)

Acid-treated fish-derived gelatin (2 parts by weight, fish gelatin FGS-230, from Nippi Inc.) as slightly water-soluble gelatin was added to purified water (100 parts by weight) heated and dissolved at a temperature of 60°C. The solution was frozen with liquid nitrogen, followed by freeze-drying in a freeze dryer (freeze dryer DC400, from Yamato Scientific Co., Ltd.). In this manner, a freeze-dried product (FD product) of the acid-treated fish-derived gelatin was provided.
The FD product of the acid-treated fish-derived gelatin (1.0 part by weight) was added to propylene glycol (9.0 parts by weight, from Wako Pure Chemical Industries, Ltd.) and dissolved therein while agitating at a temperature of 60°C. After the dissolution, a 1.0-g portion of the solution was dispensed to a 5-cm² plastic blister case (Cryomold (square type) No. 3, from Sakura Finetek Japan Co., Ltd.), and cooled at 2°C to 8°C overnight. In this manner, an edible composition was provided.

### (Examples 12 to 15)

Edible compositions were prepared respectively using the materials shown in Table 3 in the same manner as in Example 11. Instead of the acid-treated fish-derived gelatin (fish gelatin FGS-230, from Nippi Inc.) as slightly water-soluble gelatin, the following materials were used: swine-derived low molecular weight gelatin (swine bone gelatin AEP, from Nippi Inc.) as slightly water-soluble gelatin in Example 12; alkali-treated swine-derived gelatin (alkali-treated swine gelatin BP-200D, from Nippi Inc.) in Example 13; acid-treated swine-derived gelatin (acid-treated swine gelatin AP-200, from Nippi Inc.) in Example 14; and alkali-treated bovine-derived gelatin (gelatin CP-1045, from JELLICE) in Example 15.

**[Table 3]**

| Ingredient | Example [parts by weight] | | | | |
|---|---|---|---|---|---|
| | 11 | 12 | 13 | 14 | 15 |
| Acid-treated fish-derived gelatin (FD product) | 1.0 | - | - | - | - |
| Swine-derived low molecular weight gelatin (FD product) | - | 1.0 | - | - | - |
| Alkali-treated swine-derived gelatin (FD product) | - | - | 1.0 | - | - |
| Acid-treated swine-derived gelatin (FD product) | - | - | - | 1.0 | - |
| Alkali-treated bovine-derived gelatin (FD product) | - | - | - | - | 1.0 |
| Propylene glycol | 9.0 | 9.0 | 9.0 | 9.0 | 9.0 |
| Dispensed amount [g/blister] | 1 | 1 | 1 | 1 | 1 |
| Size [cm2] | 5 | 5 | 5 | 5 | 5 |

### (Comparative Example 3)

Acid-treated fish-derived gelatin (1.0 part by weight, fish gelatin FGS-230, from Nippi Inc.) as slightly water-soluble gelatin was added to propylene glycol (9.0 parts by weight, from Wako Pure Chemical Industries, Ltd.) and agitated at a temperature of 60°C. The gelatin was not dissolved. Then, the mixture was heated up to 80°C and agitated but the gelatin was not dissolved. A 1-g portion of the mixture in which the precipitate remained was dispensed as it was to a 5-cm² plastic blister case (Cryomold (square type) No. 3, from Sakura Finetek Japan Co., Ltd.), and cooled at 2°C to 8°C overnight. In this manner, an edible composition was provided.

### (Comparative Examples 4 to 9)

Edible compositions were prepared respectively using the materials shown in Table 4 in the same manner as in Comparative Example 3. Instead of the acid-treated fish-derived gelatin (fish gelatin FGS-230, from Nippi Inc.) as slightly water-soluble gelatin, the following materials were used: swine-derived low molecular weight gelatin (swine bone gelatin AEP, from Nippi Inc.) in Comparative Example 4; alkali-treated swine-derived gelatin (alkali-treated swine gelatin BP-200D, from Nippi Inc.) in Comparative Example 5; acid-treated swine-derived gelatin (acid-treated swine gelatin AP-200, from Nippi Inc.) in Comparative Example 6; alkali-treated bovine-derived gelatin (gelatin CP-1045, from JELLICE) in Comparative Example 7; carboxymethyl cellulose sodium (from MP biomedicals) in Comparative Example 8; and carboxyvinyl polymer (CARBOPOL 971P, from NOVEON) in Comparative Example 9.

**[Table 4]**

| Ingredient | Comparative Example [parts by weight] | | | | | | |
|---|---|---|---|---|---|---|---|
| | 3 | 4 | 5 | 6 | 7 | 8 | 9 |
| Acid-treated fish-derived gelatin | 1.0 | - | - | - | - | - | - |
| Swine-derived low molecular weight gelatin | - | 1.0 | - | - | - | - | - |
| Alkali-treated swine-derived gelatin | - | - | 1.0 | - | - | - | - |
| Acid-treated swine-derived gelatin | - | - | - | 1.0 | - | - | - |
| Alkali-treated bovine-derived gelatin | - | - | - | - | 1.0 | - | - |
| CMCNa | - | - | - | - | - | 1.0 | - |
| Carboxyvinyl polymer | - | - | - | - | - | - | 1.0 |
| Propylene glycol | 9.0 | 9.0 | 9.0 | 9.0 | 9.0 | 9.0 | 9.0 |
| Dispensed amount [g/blister] | 1 | 1 | 1 | 1 | 1 | 1 | 1 |
| Size [cm2] | 5 | 5 | 5 | 5 | 5 | 5 | 5 |

### (Comparative Example 10)

Acid-treated fish-derived gelatin (1.0 part by weight, fish gelatin FGS-230, from Nippi Inc.) as slightly water-soluble gelatin was added to glycerin (9.0 parts by weight, from Wako Pure Chemical Industries, Ltd.) and agitated at a temperature of 60°C. The gelatin was not dissolved. Then, the mixture was heated up to 80°C and agitated but the gelatin was not dissolved. A 1-g portion of the mixture in which the precipitate remained was dispensed as it was to a 5-cm² plastic blister case (Cryomold (square type) No. 3, from Sakura Finetek Japan Co., Ltd.), and cooled at 2°C to 8°C overnight. In this manner, an edible composition was provided.

### (Comparative Examples 11 to 16)

Edible compositions were prepared respectively using the materials shown in Table 5 in the same manner as in Comparative Example 10. Instead of the acid-treated fish-derived gelatin (fish gelatin FGS-230, from Nippi Inc.) as slightly water-soluble gelatin, the following materials were used: swine-derived low molecular weight gelatin (swine bone gelatin AEP, from Nippi Inc.) in Comparative Example 11; alkali-treated swine-derived gelatin (alkali-treated swine gelatin BP-200D, from Nippi Inc.) in Comparative Example 12; acid-treated swine-derived gelatin (acid-treated swine gelatin AP-200, from Nippi Inc.) in Comparative Example 13; alkali-treated bovine-derived gelatin (gelatin CP-1045, from JELLICE) in Comparative Example 14; carboxymethyl cellulose sodium (from MP biomedicals) in Comparative Example 15; and carboxyvinyl polymer (CARBOPOL 971P, from NOVEON) in Comparative Example 16.

**[Table 5]**

| Ingredient | Comparative Example [parts by weight] | | | | | | |
|---|---|---|---|---|---|---|---|
| | 10 | 11 | 12 | 13 | 14 | 15 | 16 |
| Acid-treated fish-derived gelatin | 1.0 | - | - | - | - | - | - |
| Swine-derived low molecular weight gelatin | - | 1.0 | - | - | - | - | - |
| Alkali-treated swine-derived gelatin | - | - | 1.0 | - | - | - | - |
| Acid-treated swine-derived gelatin | - | - | - | 1.0 | - | - | - |
| Alkali-treated bovine-derived gelatin | - | - | - | - | 1.0 | - | - |
| CMCNa | - | - | - | - | - | 1.0 | - |
| Carboxyvinyl polymer | - | - | - | - | - | - | 1.0 |
| Glycerin | 9.0 | 9.0 | 9.0 | 9.0 | 9.0 | 9.0 | 9.0 |

### (Example 16)

Acid-treated fish-derived gelatin (2 parts by weight, fish gelatin FGS-230, from Nippi Inc.) as slightly water-soluble gelatin was added to purified water (100 parts by weight) heated and dissolved at a temperature of 60°C. The solution was frozen with liquid nitrogen, followed by freeze-drying in a freeze dryer (freeze dryer DC400, from Yamato Scientific Co., Ltd.). In this manner, a freeze-dried product (FD product) of the acid-treated fish-derived gelatin was provided.
The FD product of the acid-treated fish-derived gelatin (1.0 part by weight) was added to glycerin (9.0 parts by weight, from Wako Pure Chemical Industries, Ltd.) and dissolved therein while agitating at a temperature of 60°C. After the dissolution, a 1.0-g portion of the solution was dispensed to a 5-cm² plastic blister case (Cryomold (square type) No. 3, from Sakura Finetek Japan Co., Ltd.), and cooled at 2°C to 8°C overnight. In this manner, an edible composition was provided.

### (Examples 17 to 20)

Edible compositions were prepared respectively using the materials shown in Table 6 in the same manner as in Example 16. Instead of the acid-treated fish-derived gelatin (fish gelatin FGS-230, from Nippi Inc.) as slightly water-soluble gelatin, the following materials were used: swine-derived low molecular weight gelatin (swine bone gelatin AEP, from Nippi Inc.) in Example 17; alkali-treated swine-derived gelatin (alkali-treated swine gelatin BP-200D, from Nippi Inc.) in Example 18; acid-treated swine-derived gelatin (acid-treated swine gelatin AP-200, from Nippi Inc.) in Example 19; and alkali-treated bovine-derived gelatin (gelatin CP-1045, from JELLICE) in Example 20.

**[Table 6]**

| Ingredient | Example [parts by weight] | | | | |
|---|---|---|---|---|---|
| | 16 | 17 | 18 | 19 | 20 |
| Acid-treated fish-derived gelatin (FD product) | 1.0 | - | - | - | - |
| Swine-derived low molecular weight gelatin (FD product) | - | 1.0 | - | - | - |
| Alkali-treated swine-derived gelatin (FD product) | - | - | 1.0 | - | - |
| Acid-treated swine-derived gelatin (FD product) | - | - | - | 1.0 | - |
| Alkali-treated bovine-derived gelatin (FD product) | - | - | - | - | 1.0 |
| Glycerin | 9.0 | 9.0 | 9.0 | 9.0 | 9.0 |

### (Example 21)

Swine-derived low molecular weight gelatin (1 part by weight, swine bone gelatin AEP, from Nippi Inc.) as slightly water-soluble gelatin was added to purified water (2 parts by weight) and heated and dissolved at a temperature of 60°C.
To the solution was added propylene glycol (9.0 parts by weight, from Wako Pure Chemical Industries, Ltd.) and agitated at a temperature of 60°C. Thereafter, a 1.2-g portion of the mixture was dispensed to a 5-cm² plastic blister case (Cryomold (square type) No. 3, from Sakura Finetek Japan Co., Ltd.), and cooled in a desiccator having a molecular sieve (from Wako Pure Chemical Industries, Ltd.) at 2°C to 8°C for three days. In this manner, an edible composition was provided.

### (Examples 22 to 28)

Edible compositions were prepared respectively using the materials shown in Table 7 in the same manner as in Example 21. Instead of the swine-derived low molecular weight gelatin (swine bone gelatin AEP, from Nippi Inc.) as slightly water-soluble gelatin, the following materials were used: alkali-treated swine-derived gelatin (alkali-treated swine gelatin BP-200D, from Nippi Inc.) in Examples 22 and 26; acid-treated swine-derived gelatin (acid-treated swine gelatin AP-200, from Nippi Inc.) in Examples 23 and 27; and alkali-treated bovine-derived gelatin (gelatin CP-1045, from JELLICE) in Examples 24 and 28. In Examples 25 to 28, glycerin (from Wako Pure Chemical Industries, Ltd.) was used instead of propylene glycol (from Wako Pure Chemical Industries, Ltd.).

**[Table 7]**

| Ingredient | Example [parts by weight] | | | | | | | |
|---|---|---|---|---|---|---|---|---|
| | 21 | 22 | 23 | 24 | 25 | 26 | 27 | 28 |
| Swine-derived low molecular weight gelatin | 1.0 | - | - | - | 1.0 | - | - | - |
| Alkali-treated swine-derived gelatin | - | 1.0 | - | - | - | 1.0 | - | - |
| Acid-treated swine-derived gelatin | - | - | 1.0 | - | - | - | 1.0 | - |
| Alkali-treated bovine-derived gelatin | - | - | - | 1.0 | - | - | - | 1.0 |
| Purified water | (2.0) | (2.0) | (2.0) | (2.0) | (2.0) | (2.0) | (2.0) | (2.0) |
| Propylene glycol | 9.0 | 9.0 | 9.0 | 9.0 | - | - | - | - |
| Glycerin | - | - | - | - | 9.0 | 9.0 | 9.0 | 9.0 |

### (Example 29)

Swine-derived low molecular weight gelatin (2 parts by weight, swine bone gelatin AEP, from Nippi Inc.) as slightly water-soluble gelatin was added to purified water (100 parts by weight) heated and dissolved at a temperature of 60°C. The solution was frozen with liquid nitrogen, followed by freeze-drying in a freeze dryer (freeze dryer DC400, from Yamato Scientific Co., Ltd.). In this manner, a freeze-dried product (FD product) of the swine-derived low molecular weight gelatin was provided.
The FD product of the swine-derived low molecular weight gelatin (5 parts by weight) was added to glycerin (43.9 parts by weight, from Wako Pure Chemical Industries, Ltd.) heated and dissolved therein at a temperature of 60°C. To the solution was added precipitated calcium carbonate (50 parts by weight, from Bihoku Funka Kogyo Co., Ltd.) as a drug, sucralose (0.1 parts by weight, from San-Ei Gen F.F.I., Inc.), and citric acid (1.0 part by weight, citric acid hydrate (Japanese Pharmacopoeia) from Komatsuya Corporation) and agitated at a temperature of 50°C. Thereafter, a 2.0-g portion of the solution was dispensed to a 5-cm² plastic blister case (Cryomold (square type) No. 3, from Sakura Finetek Japan Co., Ltd.), and cooled at 2°C to 8°C overnight. In this manner, a film preparation was provided.

### (Examples 30 and 31)

Film preparations were prepared respectively using the materials shown in Table 8 in the same manner as in Example 29. Instead of the precipitated calcium carbonate (from Bihoku Funka Kogyo Co., Ltd.) as the drug, lanthanum carbonate hydrate (from Wako Pure Chemical Industries, Ltd.) was used in Example 30, and ethyl icosapentate (from Wako Pure Chemical Industries, Ltd.) was used in Example 31.

**[Table 8]**

| Ingredient | Example [parts by weight] | | |
|---|---|---|---|
| | 29 | 30 | 31 |
| Precipitated calcium carbonate | 50.0 | - | - |
| Lanthanum carbonate hydrate | - | 40.0 | - |
| Ethyl icosapentate | - | - | 30.0 |
| Swine-derived low molecular weight gelatin (FD product) | 5.0 | 6.0 | 7.0 |
| Sucralose | 0.1 | 0.1 | 0.1 |
| Citric acid | 1.0 | 1.0 | 1.0 |
| Glycerin | 43.9 | 52.9 | 60.9 |

### [Test method]

The edible compositions or film preparations (hereinafter, collectively referred to as sample) in the examples and the comparative examples were evaluated for the following three properties: whether the respective gelling agents were dissolved upon preparation of the samples (preparability); whether the samples became a jelly by cooling after the preparation (gelling property); and whether separation of the nonvolatile organic solvents occurred after one month storage at a temperature of 25°C (storage stability). Table 9 shows the results.
Disintegration test was performed on the samples of Examples 1 to 7 and 11 to 20 so as to examine the dissolution property in the mouth, and the dissolution time was measured. Table 10 shows the results.
The methods for the respective tests are described below.

### (1) Preparability

The samples were evaluated on whether the gelling agents were dissolved in the respective nonvolatile organic solvents upon preparation of the samples. The scoring criteria are as follows.
3: The sample was completely dissolved to be a transparent solution.
2: The sample was dissolved, but a little turbidity was observed.
1: A little insoluble residue remained.
0: The sample was dissolved only very slightly.

### (2) Gelling property (Jellifying property)

The samples were evaluated on whether they became a jelly by cooling at a temperature of 2°C to 8°C after preparation of the samples. The scoring criteria are as follows:
3: The sample became an edible jelly composition or jelly preparation which was elastic upon pressed by a finger, leaving no gelling agent on the finger.
2: The sample became an edible jelly composition or jelly preparation, being soft with insufficient elasticity.
1: The sample became an edible jelly composition or jelly preparation, leaving the gelling agent on the finger.
0: The sample was not gelatinized at all by the viscous gelling agent.

### (3) Storage stability test

The prepared samples were stored in a constant-temperature bath set to 25°C and taken out of the bath one. month after the start of the storage. The samples were evaluated by a sensory test (texture). The evaluation was performed based on the evaluation method of a sensory test (texture).
3: No separation of the nonvolatile organic solvent (liquid component) was observed.
2: A little separation of the nonvolatile organic solvent (liquid component) was observed on the surface of the sample.
1: Considerable separation of the nonvolatile organic solvent (liquid component) was observed on the surface of the sample.
0: The nonvolatile organic solvent (liquid component) was separated greatly, and the gelling agent was soaked therein.
The above test was not performed on the samples which were scored "0: The sample was dissolved only very slightly" in the evaluation of the preparability (1) and the samples scored "0: The sample was not gelatinized at all by the viscous gelling agent" in the evaluation of the gelling property (2), and those samples were scored "0".

### (4) Measurement of dissolution time in the mouth

A disintegration test was performed according to the 15^{th} revision of Japanese pharmacopoeia. The test was performed under the following condition: Distilled water was placed in a 1000 mL low-type beaker of a test apparatus, and then the test apparatus was reciprocated up and down 29 to 32 times per minute with an amplitude of 53 to 57 mm at a temperature of 37±2°C.
The edible composition of each of Examples 1 to 7 and 11 to 20 was placed in the test apparatus, and the test was performed under the aforementioned condition. The time period from the start of the test until the edible composition of each of Examples 1 to 7 and 11 to 20 was completely dissolved and disappeared in the test apparatus was determined as a dissolution time in the mouth.

**[Table 9]**

| Sample | Preparability | Gelling property (Jellifying property) | Storage stability | Evaluation |
|---|---|---|---|---|
| Example 1 | 3 | 2 | 3 | 8 |
| Example 2 | 3 | 3 | 3 | 9 |
| Example 3 | 3 | 3 | 3 | 9 |
| Example 4 | 3 | 3 | 3 | 9 |
| Example 5 | 3 | 3 | 3 | 9 |
| Example 6 | 3 | 3 | 3 | 9 |
| Example 7 | 3 | 3 | 3 | 9 |
| Example 8 | 3 | 3 | 3 | 9 |
| Example 9 | 3 | 3 | 3 | 9 |
| Example 10 | 3 | 3 | 3 | 9 |
| Example 11 | 3 | 3 | 3 | 9 |
| Example 12 | 3 | 3 | 3 | 9 |
| Example 13 | 3 | 3 | 3 | 9 |
| Example 14 | 3 | 3 | 2 | 8 |
| Example 15 | 3 | 3 | 3 | 9 |
| Example 16 | 3 | 3 | 3 | 9 |
| Example 17 | 3 | 3 | 3 | 9 |
| Example 18 | 3 | 3 | 3 | 9 |
| Example 19 | 3 | 3 | 3 | 9 |
| Example 20 | 3 | 3 | 3 | 9 |
| Example 21 | 3 | 3 | 3 | 9 |
| Example 22 | 3 | 3 | 3 | 9 |
| Example 23 | 3 | 3 | 2 | 8 |
| Example 24 | 3 | 3 | 3 | 9 |
| Example 25 | 3 | 3 | 3 | 9 |
| Example 26 | 3 | 3 | 3 | 9 |
| Example 27 | 3 | 3 | 3 | 9 |
| Example 28 | 3 | 3 | 3 | 9 |
| Example 29 | 3 | 3 | 3 | 9 |
| Example 30 | 3 | 3 | 3 | 9 |
| Example 31 | 3 | 3 | 3 | 9 |
| Comparative Example 1 | 3 | 0 | 0 | 3 |
| Comparative Example 2 | 1 | 1 | 1 | 3 |
| Comparative Example 3 | 1 | 2 | 3 | 6 |
| Comparative Example 4 | 1 | 2 | 2 | 5 |
| Comparative Example 5 | 1 | 2 | 3 | 6 |
| Comparative Example 6 | 1 | 2 | 2 | 5 |
| Comparative Example 7 | 1 | 1 | 3 | 5 |
| comparative Example 8 | 3 | 0 | 0 | 3 |
| Comparative Example 9 | 3 | 0 | 0 | 3 |
| Comparative Example 10 | 1 | 2 | 3 | 6 |
| Comparative Example 11 | 1 | 2 | 3 | 6 |
| Comparative Example 12 | 1 | 2 | 3 | 6 |
| Comparative Example 13 | 1 | 2 | 2 | 5 |
| Comparative Example 14 | 1 | 1 | 3 | 5 |
| Comparative Example 15 | 3 | 0 | 0 | 3 |
| Comparative Example 16 | 3 | 0 | 0 | 3 |

As shown in Table 9, the samples of the examples were water-free edible jelly compositions or jelly preparations and achieved good results in all the evaluation items. The samples achieved good evaluation results as well even when various gelling agents (freely water soluble gelatin) were respectively added. Moreover, the samples also achieved good evaluation results even when various nonvolatile organic solvents (glycerin and propylene glycol) were respectively used.
In contrast, the sample of Comparative Example 1 had poor result concerning the gelling property (jellifying property). The sample of Comparative Example 2 had poor results in all the evaluation items. The samples of Comparative Examples 3 to 7 and 10 to 14 showed good storage stability but had poor results concerning the preparability and gelling property (jellifying property). The samples of Comparative Examples 8, 9, 15 and 16 showed good preparability but had poor results concerning the gelling property (jellifying property) and storage stability.

**[Table 10]**

| Sample | Dissolution time [sec] |
|---|---|
| Example 1 | 39 |
| Example 2 | 118 |
| Example 3 | 157 |
| Example 4 | 226 |
| Example 5 | 144 |
| Example 6 | 141 |
| Example 7 | 156 |
| Example 11 | 84 |
| Example 12 | 123 |
| Example 13 | 181 |
| Example 14 | 110 |
| Example 15 | 186 |
| Example 16 | 111 |
| Example 17 | 131 |
| Example 18 | 177 |
| Example 19 | 152 |
| Example 20 | 193 |

As shown in Table 10, the dissolution time in the mouth could be adjusted by controlling the amount of the nonvolatile organic solvent in the edible jelly composition.

### (Example 32)

To purified water (10.0 parts by weight, Japanese pharmacopoeia purified water, from Kenei Pharmaceutical Co., Ltd.) was added κ-carrageenan (0.5 parts by weight, GENUGEL JPE-126, from CP Kelco), and agitated sufficiently. Thereafter, propylene glycol (9.5 parts by weight, from Wako Pure Chemical Industries, Ltd.) was added thereto, and dissolved while agitation at a temperature of 85°C. After the dissolution, a 2.0-g portion of the solution was dispensed to a 5-cm² plastic blister case (Cryomold (square type) No. 3, from Sakura Finetek Japan Co., Ltd.), and dried at a temperature of 40°C for three hours in a vacuum dryer (DP63, from Yamato Scientific Co., Ltd.) so that water was completely evaporated. In this manner, an edible composition was provided.

### (Examples 33 to 37)

Edible compositions were prepared respectively using the materials shown in Table 11 in the same manner as in Example 32. Instead of the κ-carrageenan, the following materials were used: xanthan gum (Rhaball gum GS-C, from DSP Gokyo Food & Chemical Co., Ltd.) in Example 33; deacylated gellan gum (Kelcogel, from CP Kelco) in Example 34; tamarind gum (Glyloid 3S, from DSP Gokyo Food & Chemical Co., Ltd.) in Example 35; LM pectin (GENU PECTIN LM-102AS-J, from CP Kelco) in Example 36; and HM pectin (GENU PECTIN USP-H, from CP Kelco) in Example 37.

### (Comparative Example 17)

To purified water (10.0 parts by weight, Japanese pharmacopoeia purified water, from Kenei Pharmaceutical) was added 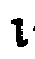 -carrageenan (0.5 parts by weight, GENUVISCO PJ-JPE, from CP Kelco), and agitated sufficiently. Thereafter, propylene glycol (9.5 parts by weight, from Wako Pure Chemical Industries, Ltd.) was added thereto, and dissolved while agitation at a temperature of 85°C. After the dissolution, a 2.0-g portion of the solution was dispensed to a 5-cm² plastic blister case (Cryomold (square type) No. 3, from Sakura Finetek Japan Co., Ltd.), and dried at a temperature of 40°C for three hours in a vacuum dryer (DP63, from Yamato Scientific Co., Ltd.) so that water was completely evaporated. In this manner, an edible composition was provided.

### (Comparative Examples 18 to 23)

Edible compositions were prepared respectively using the materials shown in Table 11 in the same manner as in Comparative Example 17. Instead of the 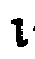 -carrageenan, the following materials were used: λ-carrageenan (ML200, from MRC Polysaccharide Co., Ltd.) in Comparative Example 18; locust bean gum (GENUGUM RL-200-J, from CP Kelco) in Comparative Example 19; native-type gellan gum (Kelcogel LT100, from CP Kelco) in Comparative Example 20; guar gum (MEYORO-GUAR CSA200/50, from DANISCO) in Comparative Example 21; carboxymethyl cellulose sodium (CMCNa, from MP Biomedicals) in Comparative Example 22; and carboxyvinyl polymer (Carbopol 971P, from NOVEON) in Comparative Example 23.

**[Table 11]**

| Ingredient | Example [parts by weight] | Comparative Example [parts by weight] | | | Example [parts by weight] | Comparative Example [parts by weight] | Example [parts by weight] | | | | Comparative Example [parts by weight] | | |
|---|---|---|---|---|---|---|---|---|---|---|---|---|---|
| | 32 | 17 | 18 | 19 | 33 | 20 | 34 | 35 | 36 | 37 | 21 | 22 | 23 |
| κ-Caffageenan | 0.5 | - | - | - | - | - | - | . | - | - | - | - | - |
| 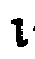 -Carrageenan | - | 0.5 | - | - | - | - | - | - | - | - | - | - | - |
| λ-Cartageenan | - | - | 0.5 | - | - | - | - | - | - | - | - | - | - |
| Locust bean gum | - | - | - | 0.5 | - | - | - | - | - | - | - | - | - |
| Xanthan gum | - | - | - | - | 0.5 | - | - | - | - | - | - | - | - |
| Native-type gellan gum | - | - | - | - | - | 0.5 | - | - | - | - | - | - | - |
| Deacylated gellan gum | - | - | - | - | - | - | 0.5 | - | - | - | - | - | - |
| Tamarind gum | - | - | - | - | - | - | - | 0.5 | - | - | - | - | - |
| LM pectin | - | - | - | - | - | - | - | - | 0.5 | - | - | - | - |
| HM pectin | - | - | - | - | - | - | - | - | - | 0.5 | - | - | - |
| Guar gum | - | - | - | - | - | - | - | - | - | - | 0.5 | - | - |
| CMCNa | - | - | - | - | - | - | - | - | - | - | - | 0.5 | - |
| Carboxyvinyl polymer | - | - | - | - | - | - | - | - | - | - | - | - | 0.5 |
| Purified water | (10.0) | (10.0) | (10.0) | (10.0) | (10.0) | (10.0) | (10.0) | (10.0) | (10.0) | (10.0) | (10.0) | (10.0) | (10.0) |
| Propylene glycol | 9.5 | 9.5 | 9.5 | 9.5 | 9.5 | 9.5 | 9.5 | 9.5 | 9.5 | 9.5 | 9.5 | 9.5 | 9.5 |
| Dispensed amount (g/blister) | 20 | 20 | 20 | 20 | 20 | 20 | 20 | 20 | 20 | 20 | 20 | 20 | 20 |

### (Example 38)

To purified water (10.0 parts by weight, Japanese pharmacopoeia purified water, from Kenei Pharmaceutical) was added κ-carrageenan (0.5 parts by weight, GENUGEL JPE-126, from CP Kelco), and agitated sufficiently. Thereafter, glycerin (9.5 parts by weight, from Wako Pure Chemical Industries, Ltd.) was added thereto, and dissolved while agitation at a temperature of 85°C. After the dissolution, a 2.0-g portion of the solution was dispensed to a 5-cm² plastic blister case (Cryomold (square type) No. 3, from Sakura Finetek Japan Co., Ltd.), and dried at a temperature of 40°C for three hours in a vacuum dryer (DP63, from Yamato Scientific Co., Ltd.) so that water was completely evaporated. In this manner, an edible composition was provided.

### (Examples 39 to 42)

Edible compositions were prepared respectively using the materials shown in Table 12 in the same manner as in Example 38. Instead of the κ-carrageenan, the following materials were used: native-type gellan gum (Kelcogel LT100, from CP Kelco) in Example 39; deacylated gellan gum (Kelcogel, from CP Kelco) in Example 40; tamarind gum (Glyloid 3S, from DSP Gokyo Food & Chemical Co., Ltd.) in Example 41; and LM pectin (GENU PECTIN LM-102AS-J, from CP Kelco) in Example 42.

### (Comparative Example 24)

To purified water (10.0 parts by weight, Japanese pharmacopoeia purified water, from Kenei Pharmaceutical) was added τ-carrageenan (0.5 parts by weight, GENUVISCO PJ-JPE, from CP Kelco), and agitated sufficiently. Thereafter, glycerin (9.5 parts by weight, from Wako Pure Chemical Industries, Ltd.) was added thereto, and dissolved while agitation at a temperature of 85°C. After the dissolution, a 2.0-g portion of the solution was dispensed to a 5-cm² plastic blister case (Cryomold (square type) No. 3, from Sakura Finetek Japan Co., Ltd.), and dried at a temperature of 40°C for three hours in a vacuum dryer (DP63, from Yamato Scientific Co., Ltd.) so that water was completely evaporated. In this manner, an edible composition was provided.

### (Comparative Examples 25 to 31)

Edible compositions were prepared respectively using the materials shown in Table 12 in the same manner as in Comparative Example 24. Instead of the τ-carrageenan, the following materials were used: λ-carrageenan (ML200, from MRC Polysaccharide Co., Ltd.) in Comparative Example 25; locust bean gum (GENUGUM RL-200-J, from CP Kelco) in Comparative Example 26; xanthan gum (Rhaball gum GS-C, from DSP Gokyo Food & Chemical Co., Ltd.) in Comparative Example 27; HM pectin (GENU PECTIN USP-H, from CP Kelco) in Comparative Example 28; guar gum (MEYORO-GUAR CSA200/50, from DANISCO) in Comparative Example 29; carboxymethyl cellulose sodium (CMCNa, from MP Biomedicals) in Comparative Example 30; and carboxyvinyl polymer (Carbopol 971P, from NOVEON) in Comparative Example 31.

**[Table 12]**

| Ingredient | Example [parts by weight] | Comparative Example [parts by weight] | | | | Example [parts by weight] | | | | Comparative Example [parts by weight] | | | |
|---|---|---|---|---|---|---|---|---|---|---|---|---|---|
| | 38 | 24 | 25 | 26 | 27 | 39 | 40 | 41 | 42 | 28 | 29 | 30 | 31 |
| κ-Canageanan | 0.5 | - | - | - | - | - | - | - | - | - | - | - | - |
| 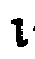 -Canageenan | - | 0.5 | - | - | - | - | - | - | - | - | - | - | - |
| λ-Carrageenan | - | - | 0.5 | - | - | - | - | - | - | - | - | - | - |
| Locust bean gum | - | - | - | 0.5 | - | - | - | - | - | - | - | - | - |
| Xanthan gum | - | - | - | - | 0.5 | - | - | - | - | - | - | - | - |
| Native-type gellan gum | - | - | - | - | - | 0.5 | - | - | - | - | - | - | - |
| Deacylated gellan gum | - | - | - | - | - | - | 0.5 | - | . | - | - | - | - |
| Tamarind gum | - | - | - | - | - | - | - | 0.5 | - | - | - | - | - |
| LM pectin | - | - | - | - | - | - | - | - | 0.5 | - | - | - | - |
| HM pectin | - | - | - | - | - | - | - | - | - | 0.5 | - | - | - |
| Guar gum | - | - | - | - | - | - | - | - | - | - | 0.5 | - | - |
| CMCNa | - | - | - | - | - | - | - | - | - | - | - | 0.5 | - |
| Carboxyvinyl polymer | - | - | - | - | - | - | - | - | - | - | - | - | 0.5 |
| Purified water | (10.0) | (10.0) | (10.0) | (10.0) | (10.0) | (10.0) | (10.0) | (10.0) | (10.0) | (10.0) | (10.0) | (10.0) | (10.0) |
| Glycerin | 9.5 | 9.5 | 9.5 | 9.5 | 9.5 | 9.5 | 9.5 | 9.5 | 9.5 | 9.5 | 9.5 | 9.5 | 9.5 |
| Dispensed amount [g/blister] | 20 | 2.0 | 20 | 20 | 20 | 20 | 20 | 20 | 20 | 20 | 20 | 2.0 | 20 |

### (Comparative Example 32)

To purified water (10.0 parts by weight, Japanese pharmacopoeia purified water, from Kenei Pharmaceutical) was added κ-carrageenan (0.5 parts by weight, GENUGEL JPE-126, from CP Kelco), and agitated sufficiently. Thereafter, polyethylene glycol 400 (9.5 parts by weight, from Wako Pure Chemical Industries, Ltd.) was added thereto, and dissolved while agitation at a temperature of 85°C. After the dissolution, a 2.0-g portion of the solution was dispensed to a 5-cm² plastic blister case (Cryomold (square type) No. 3, from Sakura Finetek Japan Co., Ltd.), and dried at a temperature of 40°C for three hours in a vacuum dryer (DP63, from Yamato Scientific Co., Ltd.) so that water was completely evaporated. In this manner, an edible composition was provided.

### (Comparative Examples 33 to 44)

Edible compositions were prepared respectively using the materials shown in Table 13 in the same manner as in Comparative Example 32. Instead of the κ-carrageenan, the following materials were used: 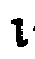 -carrageenan (GENUVISCO PJ-JPE, from CP Kelco) in Comparative Example 33; λ-carrageenan (Soageena ML200, from MRC Polysaccharide Co., Ltd.) in Comparative Example 34; locust bean gum (GENUGUM RL-200-J, from CP Kelco) in Comparative Example 35; xanthan gum (Rhaball gum GS-C, from DSP Gokyo Food & Chemical Co., Ltd.) in Comparative Example 36; native-type gellan gum (Kelcogel LT100, from CP Kelco) in Comparative Example 37; deacylated gellan gum (Kelcogel, from CP Kelco) in Comparative Example 38; tamarind gum (Glyloid 3S, from DSP Gokyo Food & Chemical Co., Ltd.) in Comparative Example 39; LM pectin (GENU PECTIN LM-102AS-J, from CP Kelco) in Comparative Example 40; HM pectin (GENU PECTIN USP-H, from CP Kelco) in Comparative Example 41; guar gum (MEYORO-GUAR CSA200/50, from DANISCO) in Comparative Example 42; carboxymethyl cellulose sodium (CMCNa, from MP Biomedicals) in Comparative Example 43; and carboxyvinyl polymer (Carbopol 971P, from NOVEON) in Comparative Example 44.

**[Table 13]**

| Ingredient | Comparative Example [parts by weight] | | | | | | | | | | | | |
|---|---|---|---|---|---|---|---|---|---|---|---|---|---|
| | 32 | 33 | 34 | 35 | 36 | 37 | 38 | 39 | 40 | 41 | 42 | 43 | 44 |
| κ-Carrageenan | 0.5 | - | - | - | - | - | - | - | - | - | - | - | - |
| 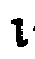 -Carnlgeenan | - | 0.5 | - | - | - | - | - | - | - | - | - | - | - |
| λ-Carrageenan | - | - | 0.5 | - | - | - | - | - | - | - | - | - | - |
| Locust bean gum | - | - | - | 0.5 | - | - | - | - | - | - | - | - | - |
| Xanthan gum | - | - | - | - | 0.5 | - | - | - | - | - | - | - | - |
| Native-type gellan gum | - | - | - | - | - | 0.5 | - | - | - | - | - | - | - |
| Deacylated gellan gum | - | - | - | - | - | - | 0.5 | - | - | - | - | - | - |
| Tamarind gum | - | - | - | - | - | - | - | 0.5 | - | - | - | - | - |
| LM pectin | - | - | - | - | - | - | - | - | 0.5 | - | - | - | - |
| HM pectin | - | - | - | - | - | - | - | - | - | 0.5 | - | - | - |
| Guar gum | - | - | - | - | - | - | - | - | - | - | 0.5 | - | - |
| CMCNa | - | - | - | - | - | - | - | - | - | - | - | 0.5 | - |
| Carboxyvinyl polymer | - | - | - | - | - | - | - | - | - | - | - | - | 0.5 |
| Purified water | (10.0) | (10.0) | (10.0) | (10.0) | (10.0) | (10.0) | (10.0) | (10.0) | (10.0) | (10.0) | (10.0) | (10.0) | (10.0) |
| Polyethylene glycol 400 | 9.5 | 9.5 | 9.5 | 9.5 | 9.5 | 9.5 | 9.5 | 9.5 | 9.5 | 9.5 | 9.5 | 9.5 | 9.5 |
| Dispensed amount [g/blister] | 2.0 | 2.0 | 2.0 | 2.0 | 2.0 | 2.0 | 2.0 | 2.0 | 2.0 | 2.0 | 2.0 | 2.0 | 2.0 |

### (Example 43)

To purified water (1000 parts by weight, Japanese pharmacopoeia purified water, from Kenei Pharmaceutical) was added κ-carrageenan (5 parts by weight, GENUGEL JPE-126, from CP Kelco), and dissolved while heating at a temperature of 70°C, followed by standing to cool at a room temperature. The resulting solution was frozen with liquid nitrogen, and freeze-dried in a freeze dryer (freeze dryer DC400, from Yamato Scientific Co., Ltd.) overnight.
The freeze-dried product (FD product) of the κ-carrageenan (0.5 parts by weight) was sufficiently agitated and then added to propylene glycol (9.5 parts by weight, from Wako Pure Chemical Industries, Ltd.), and dissolved while agitation at a temperature of 85°C. After the dissolution, a 1.0-g portion of the solution was dispensed to a 5-cm² plastic blister case (Cryomold (square type) No. 3, from Sakura Finetek Japan Co., Ltd.), and allowed to cool at a room temperature. In this manner, an edible composition was provided.

### (Examples 44 to 48)

Edible compositions were prepared respectively using the materials shown in Table 14 in the same manner as in Example 43. Instead of the κ-carrageenan, the following materials were used: xanthan gum (Rhaball gum GS-C, from DSP Gokyo Food & Chemical Co., Ltd.) in Example 44; deacylated gellan gum (Kelcogel, from CP Kelco) in Example 45; tamarind gum (Glyloid 3S, from DSP Gokyo Food & Chemical Co., Ltd.) in Example 46; LM pectin (GENU PECTIN LM-102AS-J, from CP Kelco) in Example 47; and HM pectin (GENU PECTIN USP-H, from CP Kelco) in Example 48.

**[Table 14]**

| Ingredient | Example [parts by weight] | | | | | |
|---|---|---|---|---|---|---|
| | 43 | 44 | 45 | 46 | 47 | 48 |
| κ-Carrageenan (FD product) | 0.5 | - | - | - | - | - |
| Xanthan gum (FD product) | - | 0.5 | - | - | - | - |
| Deacylated gellan gum (FD product) | - | - | 0.5 | - | - | - |
| Tamarind gum (FD product) | - | - | - | 0.5 | - | - |
| LM pectin (FD product) | - | - | - | - | 0.5 | - |
| HM pectin (FD product) | - | - | - | - | - | 0.5 |
| Propylene glycol | 9.5 | 9.5 | 9.5 | 9.5 | 9.5 | 9.5 |
| Dispensed amount [g/blister] | 1.0 | 1.0 | 1.0 | 1.0 | 1.0 | 1.0 |

### (Example 49)

To purified water (1000 parts by weight, Japanese pharmacopoeia purified water, from Kenei Pharmaceutical) was added κ-carrageenan (5 parts by weight, GENUGEL JPE-126, from CP Kelco), and dissolved while heating at a temperature of 70°C, followed by standing to cool at a room temperature. The resulting solution was frozen with liquid nitrogen, and freeze-dried in a freeze dryer (freeze dryer DC400, from Yamato Scientific Co., Ltd.) overnight.
The freeze-dried product (FD product) of the κ-carrageenan (0.5 parts by weight) was sufficiently agitated and then added to glycerin (9.5 parts by weight, from Wako Pure Chemical Industries, Ltd.), and dissolved while agitation at a temperature of 85°C. After the dissolution, a 1.0-g portion of the solution was dispensed to a 5-cm² plastic blister case (Cryomold (square type) No. 3, from Sakura Finetek Japan Co., Ltd.), and allowed to cool at a room temperature. In this manner, an edible composition was provided.

### (Examples 50 to 53)

Edible compositions were prepared respectively using the materials shown in Table 15 in the same manner as in Example 49. Instead of the κ-carrageenan, the following materials were used: native-type gellan gum (Kelcogel LT100, from CP Kelco) in Example 50; deacylated gellan gum (Kelcogel, from CP Kelco) in Example 51; tamarind gum (Glyloid 3S, from DSP Gokyo Food & Chemical Co., Ltd.) in Example 52; and LM pectin (GENU PECTIN LM-102AS-J, from CP Kelco) in Example 53.

**[Table 15]**

| Ingredient | Example [parts by weight] | | | | |
|---|---|---|---|---|---|
| | 49 | 50 | 51 | 52 | 53 |
| κ-Carrageenan (FD product) | 0.5 | - | - | - | - |
| Native-type gellan gum (FD product) | - | 0.5 | - | - | - |
| Deacylated gellan gum (FD product) | - | - | 0.5 | - | - |
| Tamarind gum (FD product) | - | - | - | 0.5 | - |
| LM pectin (FD product) | - | - | - | - | 0.5 |
| Glycerin | 9.5 | 9.5 | 9.5 | 9.5 | 9.5 |
| Dispensed amount [g/blister] | 1.0 | 1.0 | 1.0 | 1.0 | 1.0 |

### (Example 54)

To purified water (10.0 parts by weight, Japanese pharmacopoeia purified water, from Kenei Pharmaceutical) was added κ-carrageenan (0.2 parts by weight, GENUGEL JPE-126, from CP Kelco), and agitated sufficiently. Thereafter, glycerin (19.8 parts by weight, from Wako Pure Chemical Industries, Ltd.) was added thereto, and dissolved while agitation at a temperature of 85°C. After the dissolution, a 1.5-g portion of the solution was dispensed to a 5-cm² plastic blister case (Cryomold (square type) No. 3, from Sakura Finetek Japan Co., Ltd.), and dried at a temperature of 40°C for five hours in a vacuum dryer (DP63, from Yamato Scientific Co., Ltd.) so that water was completely evaporated. In this manner, an edible composition was provided.

### (Examples 55 to 63)

Edible compositions were prepared respectively using the materials shown in Table 16 in the same manner as in Example 54. Instead of the glycerin, propylene glycol (from Wako Pure Chemical Industries, Ltd.) was used in Examples 59 to 63.

**[Table 16]**

| Ingredient | Example [parts by weight] | | | | | | | | | |
|---|---|---|---|---|---|---|---|---|---|---|
| | 54 | 55 | 56 | 57 | 58 | 59 | 60 | 61 | 62 | 63 |
| κ-Carrageenan | 0.2 | 0.5 | 1.0 | 1.5 | 2.0 | 0.2 | 0.5 | 1.0 | 1.5 | 20 |
| Purified water | (10.0) | (10.0) | (15.0) | (20.0) | (25.0) | (15.0) | (20.0) | (25.0) | (40.0) | (50.0) |
| Propylene glycol | - | - | - | - | - | 19.8 | 19.5 | 19.0 | 18.5 | 18.0 |
| Glycerin | 19.8 | 19.5 | 19.0 | 18.5 | 18.0 | - | - | - | - | - |
| Dispensed amount [g/blister] | 1.5 | 1.5 | 1.75 | 2.0 | 2.25 | 1.75 | 2.0 | 2.5 | 3.0 | 3.5 |

### (Example 64)

To purified water (10.0 parts by weight, Japanese pharmacopoeia purified water, from Kenei Pharmaceutical) was added xanthan gum (0.2 parts by weight, Rhaball gum GS-C, from DSP Gokyo Food & Chemical Co., Ltd.), and agitated sufficiently. Thereafter, propylene glycol (9.8 parts by weight, from Wako Pure Chemical Industries, Ltd.) was added thereto, and dissolved while agitation at a temperature of 85°C. After the dissolution, a 2.0-g portion of the solution was dispensed to a 5-cm² plastic blister case (Cryomold (square type) No. 3, from Sakura Finetek Japan Co., Ltd.), and dried at a temperature of 40°C for five hours in a vacuum dryer (DP63, from Yamato Scientific Co., Ltd.) so that water was completely evaporated. In this manner, an edible composition was provided.

### (Examples 65 to 68)

Edible compositions were prepared respectively using the materials shown in Table 17 in the same manner as in Example 64.

**[Table 17]**

| Ingredient | Example [parts by weight] | | | | |
|---|---|---|---|---|---|
| | 64 | 65 | 66 | 67 | 68 |
| Xanthan gum | 0.2 | 0.3 | 0.5 | 1.0 | 1.5 |
| Purified water | (10.0) | (10.0) | (10.0) | (20.0) | (30.0) |
| Propylene glycol | 9.8 | 9.7 | 9.5 | 9 | 8.5 |
| Dispensed amount [g/blister] | 2.0 | 2.0 | 2.0 | 3.0 | 4.0 |

### (Example 69)

To purified water (1000 parts by weight, Japanese pharmacopoeia purified water, from Kenei Pharmaceutical) was added κ-carrageenan (5 parts by weight, GENUGEL JPE-126, from CP Kelco), and dissolved while heating at a temperature of 70°C, followed by standing to cool at a room temperature. The resulting solution was frozen with liquid nitrogen, and freeze-dried in a freeze dryer (freeze dryer DC400, from Yamato Scientific Co., Ltd.) overnight.
The freeze-dried product (FD product) of the κ-carrageenan (1.0 part by weight) was added to glycerin (43.9 parts by weight, from Wako Pure Chemical Industries, Ltd.) and dissolved therein while heating at a temperature of 85°C. In this manner, a polysaccharide gelling agent solution was provided. To the polysaccharide gelling agent solution was added precipitated calcium carbonate (50 parts by weight, from Bihoku Funka Kogyo Co., Ltd.), sucralose (0.1 parts by weight, from San-Ei Gen F.F.I., Inc.), and citric acid (1.0 part by weight, citric acid hydrate (Japanese Pharmacopoeia) from Komatsuya Corporation) and agitated at a temperature of 80°C. Thereafter, a 1.0-g portion of the solution was dispensed to a 5-cm² plastic blister case (Cryomold (square type) No. 3, from Sakura Finetek Japan Co., Ltd.), and cooled at 2°C to 8°C overnight. In this manner, a film preparation was provided.

### (Example 70)

A film preparation was provided using the materials shown in Table 18 in the same manner as in Example 69. Lanthanum carbonate hydrate (from Wako Pure Chemical Industries, Ltd.) was used instead of the precipitated calcium carbonate, and a FD product of LM pectin (GENU PECTIN LM-102AS-J, from CP Kelco) that was obtained in the same manner as the FD product of the κ-carrageenan was used instead of the FD product of the κ-carrageenan.

### (Example 71)

To purified water (60.0 parts by weight, Japanese pharmacopoeia purified water, from Kenei Pharmaceutical) was added deacylated gellan gum (1.0 part by weight Kelcogel, from CP Kelco), and agitated sufficiently. Thereafter, the mixture was added to glycerin (60.9 parts by weight, from Wako Pure Chemical Industries, Ltd.), and dissolved while heating at a temperature of 85°C. In this manner, a polysaccharide gelling agent solution was provided. The polysaccharide gelling agent solution was cooled to 60°C, and then ethyl icosapentate (30.0 pars by weight, from Wako Pure Chemical Industries, Ltd.), sucralose (0.1 parts by weight, from San-Ei Gen F.F.I., Inc.), and citric acid (1.0 part by weight, citric acid hydrate (Japanese Pharmacopoeia) from Komatsuya Corporation) were added to the solution, followed by agitation at a temperature of 60°C. Thereafter, a 1.6-g portion of the solution was dispensed to a 5-cm² plastic blister case (Cryomold (square type) No. 3, from Sakura Finetek Japan Co., Ltd.), and dried at 35°C for five hours in a vacuum dryer (DP63, from Yamato Scientific Co., Ltd.) so that water was completely evaporated. In this manner, a film preparation was provided.

### (Example 72)

A film preparation was provided using the materials shown in Table 18 in the same manner as in Example 71. Instead of the ethyl icosapentate, entacapone (from Cipla), deacylated gellan gum, and also tamarind gum (Glyloid 3S, from DSP Gokyo Food & Chemical Co., Ltd.) were used.

**[Table 18]**

| ingredient | Example [parts by weight] | | | |
|---|---|---|---|---|
| | 69 | 70 | 71 | 72 |
| Precipitated calcium carbonate | 50.0 | - | - | - |
| Lanthanum carbonate hydrate | - | 40.0 | - | - |
| Ethyl icosapentate | - | - | 30.0 | - |
| Entacapone | - | - | - | 30.0 |
| κ-Carrageenan (FD product) | 1.0 | - | - | - |
| LM pectin (FD product) | - | 1.0 | - | - |
| Deacylated gellan gum | - | - | 1.0 | 0.5 |
| Tamarind gum | - | - | - | 0.5 |
| Sucralose | 0.1 | 0.1 | 0.1 | 0.1 |
| Citric acid | 1.0 | 1.0 | 1.0 | 1.0 |
| Purified water | - | - | (60.0) | (60.0) |
| Glycerin | 43.9 | 52.9 | 60.9 | 60.9 |
| Dispensed amount [g/blister] | 1.0 | 1.0 | 1.6 | 1.6 |

### [Test method]

The edible compositions or film preparations (hereinafter, also collectively referred to as sample) in Examples and Comparative Examples were evaluated for the following four properties: whether the respective polysaccharide gelling agents were dissolved upon preparation of the samples (preparability); whether the samples became a jelly by cooling after the preparation (gelling property 1); whether separation of the liquid component occurred by cooling after the preparation (gelling property 2); and whether separation of the nonvolatile organic solvents occurred after one month storage at a temperature of 40°C (storage stability). Disintegration test was performed on the samples of Examples 32 to 42 and 54 to 58 so as to examine the dissolution property in the mouth, and the dissolution time was measured. The methods for the respective tests are described below. Tables 19 to 21 show the results.

### (1) Preparability

The samples were evaluated on whether the polysaccharide gelling agents were dissolved in the respective solvents upon preparation of the samples. The scoring criteria are as follows.
3: The sample was completely dissolved to be a transparent solution.
2: The sample was dissolved, but a little turbidity was observed.
1: A little insoluble residue remained.
0: The sample was dissolved only very slightly.

### (2) Gelling property 1 (Jellifying property)

The samples were evaluated on whether they became a jelly upon preparation thereof, or upon vacuum drying or standing to cool thereof. The scoring criteria are as follows:
3: The sample became an edible jelly composition or jelly preparation which was elastic upon pressed by a finger, leaving no polysaccharide gelling agent on the finger.
2: The sample became an edible jelly composition or jelly preparation, being soft with insufficient elasticity.
1: The sample became an edible jelly composition or jelly preparation, leaving the polysaccharide gelling agent on the finger.
0: The sample was not gelatinized at all by the viscous polysaccharide gelling agent.
Meanwhile, the above test was not performed on the samples which were scored "0: The sample was dissolved only very slightly" in the evaluation of the (1) preparability, and those samples were scored "0".

### (3) Gelling property 2 (Compatibility)

The samples were evaluated on whether separation of the solvent occurred during the preparation of the samples, or upon vacuum drying or standing to cool thereof. The scoring criteria are as follows:
3: No separation of the nonvolatile organic solvent (liquid component) was observed.
2: A little separation of the nonvolatile organic solvent (liquid component) was observed on the surface of the sample.
1: Considerable separation of the nonvolatile organic solvent (liquid component) was observed on the surface of the sample.
0: The nonvolatile organic solvent (liquid component) was separated greatly, and the polysaccharide gelling agent was soaked therein.
Meanwhile, the above test was not performed on the samples which were scored "0: The sample was dissolved only very slightly" in the evaluation of the (1) preparability, and those samples were scored "0".

### (4) Storage stability test

The prepared samples were stored in a constant-temperature bath set to 40°C and taken out of the bath one month after the start of the storage. The samples were examined by a sensory test (texture) and evaluated. The evaluation method was based on that of a sensory test (texture).
3: No separation of the nonvolatile organic solvent (liquid component) was observed.
2: A little separation of the nonvolatile organic solvent (liquid component) was observed on the surface of the sample.
1: Considerable separation of the nonvolatile organic solvent (liquid component) was observed on the surface of the sample.
0: The nonvolatile organic solvent (liquid component) was separated greatly, and the polysaccharide gelling agent was soaked therein.
Meanwhile, the above test was not performed on the samples which were scored "0: The sample was dissolved only very slightly" in the evaluation of the (1) preparability and the samples scored "0: The sample was not gelatinized at all by the viscous gelling agent" in the evaluation of the (2) gelling property 1, and those samples were scored "0".

### (5) Measurement of dissolution time in the mouth

A disintegration test was performed according to the 15^{th} revision of Japanese pharmacopoeia. The test was performed under the following condition: Distilled water was placed in a 1000 mL low-type beaker of a test apparatus, and the water was equilibrated to 37±2°C, and then the test apparatus was reciprocated up and down 29 to 32 times per minute with an amplitude of 53 to 57 mm. Each sample was placed in the test apparatus, and the test was performed under the aforementioned condition. The time period from the start of the test until the sample was completely dissolved and disappeared in the test apparatus was determined as a dissolution time in the mouth.

**[Table 19]**

| Sample | Preparability | Gelling property 1 (Jelliffying property) | Gelling property 2 (Compatibility) | Storage stability | Evaluation |
|---|---|---|---|---|---|
| Example 32 | 3 | 3 | 3 | 2 | 11 |
| Example 33 | 3 | 3 | 3 | 2 | 11 |
| Example 34 | 3 | 3 | 3 | 2 | 11 |
| Example 35 | 3 | 2 | 3 | 2 | 10 |
| Example 36 | 3 | 3 | 3 | 2 | 11 |
| Example 37 | 2 | 3 | 3 | 2 | 10 |
| Example 38 | 3 | 3 | 3 | 3 | 12 |
| Example 39 | 2 | 3 | 3 | 3 | 11 |
| Example 40 | 3 | 3 | 3 | 3 | 12 |
| Example 41 | 2 | 3 | 3 | 3 | 11 |
| Example 42 | 3 | 3 | 3 | 2 | 11 |
| Example 43 | 3 | 3 | 3 | 2 | 11 |
| Example 44 | 2 | 3 | 3 | 2 | 10 |
| Example 45 | 3 | 3 | 3 | 2 | 11 |
| Example 46 | 2 | 3 | 3 | 2 | 10 |
| Example 47 | 3 | 3 | 3 | 2 | 11 |
| Example 48 | 2 | 3 | 3 | 2 | 10 |
| Example 49 | 3 | 3 | 3 | 3 | 12 |
| Example 50 | 2 | 3 | 3 | 3 | 11 |
| Example 51 | 3 | 3 | 3 | 3 | 12 |
| Example 52 | 2 | 3 | 3 | 3 | 11 |
| Example 53 | 3 | 3 | 3 | 3 | 12 |
| Example 54 | 3 | 3 | 3 | 2 | 11 |
| Example 55 | 3 | 3 | 3 | 3 | 12 |
| Example 56 | 3 | 3 | 3 | 3 | 12 |
| Example 57 | 3 | 3 | 3 | 3 | 12 |
| Example 58 | 3 | 3 | 3 | 3 | 12 |
| Example 59 | 3 | 3 | 3 | 3 | 12 |
| Example 60 | 3 | 3 | 3 | 3 | 12 |
| Example 61 | 3 | 3 | 3 | 3 | 12 |
| Example 62 | 3 | 3 | 3 | 3 | 12 |
| Example 63 | 3 | 3 | 3 | 3 | 12 |
| Example 64 | 3 | 3 | 3 | 2 | 11 |
| Example 65 | 3 | 3 | 3 | 3 | 12 |
| Example 66 | 3 | 3 | 3 | 3 | 12 |
| Example 67 | 3 | 3 | 3 | 3 | 12 |
| Example 68 | 3 | 3 | 3 | 3 | 12 |
| Example 69 | 3 | 3 | 3 | 3 | 12 |
| Example 70 | 3 | 3 | 3 | 3 | 12 |
| Example 71 | 3 | 3 | 3 | 3 | 12 |
| Example 72 | 3 | 3 | 3 | 3 | 12 |

As shown in Table 19, the samples of Examples 32 to 72 achieved good results in all the evaluation items, each having the total score of 10 to 12.

**[Table 20]**

| Sample | Preparability | Gelling property 1 (Jellifying property) | Gelling property 2 (Compatibility) | Storage stability | Evaluation |
|---|---|---|---|---|---|
| Comparative Example 17 | 3 | 1 | 2 | 0 | 6 |
| Comparative Example 18 | 3 | 0 | 0 | 0 | 3 |
| Comparative Example 19 | 3 | 1 | 1 | 0 | 5 |
| Comparative Example 20 | 1 | 1 | 1 | 0 | 3 |
| Comparative Example 21 | 2 | 1 | 1 | 0 | 4 |
| Comparative Example 22 | 3 | 0 | 0 | 0 | 3 |
| Comparative Example 23 | 3 | 0 | 0 | 0 | 3 |
| Comparative Example 24 | 3 | 1 | 2 | 1 | 7 |
| Comparative Example 25 | 3 | 0 | 0 | 0 | 3 |
| Comparative Example 26 | 3 | 1 | 1 | 0 | 5 |
| Comparative Example 27 | 3 | 1 | 2 | 1 | 7 |
| Comparative Example 28 | 2 | 1 | 2 | 2 | 7 |
| Comparative Example 29 | 2 | 1 | 2 | 0 | 5 |
| Comparative Example 30 | 3 | 0 | 0 | 0 | 3 |
| Comparative Example 31 | 3 | 0 | 0 | 0 | 3 |
| Comparative Example 32 | 3 | 1 | 0 | 0 | 4 |
| Comparative Example 33 | 3 | 1 | 0 | 0 | 4 |
| Comparative Example 34 | 3 | 1 | 0 | 0 | 4 |
| Comparative Example 35 | 2 | 1 | 0 | 0 | 3 |
| Comparative Example 36 | 3 | 1 | 0 | 0 | 4 |
| Comparative Example 37 | 1 | 1 | 0 | 0 | 2 |
| Comparative Example 38 | 3 | 1 | 0 | 0 | 4 |
| Comparative Example 39 | 2 | 1 | 0 | 0 | 3 |
| Comparative Example 40 | 3 | 1 | 0 | 0 | 4 |
| Comparative Example 41 | 1 | 1 | 0 | 0 | 2 |
| Comparative Example 42 | 3 | 1 | 0 | 0 | 4 |
| Comparative Example 43 | 3 | 0 | 0 | 0 | 3 |
| Comparative Example 44 | 3 | 0 | 0 | 0 | 3 |

As shown in Table 20, the samples of Comparative Examples 17 to 44 did not achieve good results in all the evaluation items, each having a total evaluation score of 3 to 7.

**[Table 21]**

| Sample | Dissolution time [sec] | | Sample | Dissolution time [sec] |
|---|---|---|---|---|
| Example 32 | 2099 | | Example 54 | 121 |
| Example 33 | 1771 | | Example 55 | 316 |
| Example 34 | 1417 | | Example 56 | 987 |
| Example 35 | 812 | | Example 57 | 1318 |
| Example 36 | 346 | | Example 58 | 1621 |
| Example 37 | 721 | | Example 59 | 181 |
| Example 38 | 1407 | | Example 60 | 467 |
| Example 39 | 1819 | | Example 61 | 1218 |
| Example 40 | 1118 | | Example 62 | 1585 |
| Example 41 | 1371 | | Example 63 | 2012 |
| Example 42 | 189 | | Example 64 | 318 |
| | | | Example 65 | 619 |
| | | | Example 66 | 1274 |
| | | | Example 67 | 1698 |
| | | | Example 68 | 2318 |

As shown in Table 21, the dissolution time in the mouth of the samples of the examples was adjusted by controlling the amounts of the contained nonvolatile organic solvents.

### (Example 73)

Purified water (4.0 parts by weight, Japanese pharmacopoeia purified water, from Kenei Pharmaceutical) was warmed to 40°C. Fish-derived water soluble gelatin (2.0 parts by weight, water-soluble gelatin CSF, from Nippi Inc.) was added to the water, and dissolved by sufficient agitation. Propylene glycol (4.0 parts by weight, from Wako Pure Chemical Industries, Ltd.) was added to the solution, and agitated at a temperature of 40°C. Thereafter, a 1.0-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Example 74)

Purified water (4.0 parts by weight, Japanese pharmacopoeia purified water, from Kenei Pharmaceutical) was warmed to 60°C. Swine-derived low molecular weight gelatin) (2.0 parts by weight, swine bone gelatin AEP, from Nippi Inc.) was added to the water, and dissolved by sufficient agitation. Propylene glycol (4.0 parts by weight, from Wako Pure Chemical Industries, Ltd.) was added to the solution, and agitated at a temperature of 60°C. Thereafter, a 1.0-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 75 to 77)

Edible compositions were prepared respectively using the materials shown in Table 22 in the same manner as in Example 74. Instead of the swine-derived low molecular weight gelatin, the following materials were used: acid-treated swine-derived gelatin (AP-200F, from Nippi Inc.) in Example 75; alkali-treated swine-derived gelatin (BP-200F, from Nippi Inc.) in Example 76; and alkali-treated bovine-derived gelatin (AD4, from Nippi Inc.) in Example 77.

### (Example 78)

Purified water (8.0 parts by weight, Japanese pharmacopoeia purified water, from Kenei Pharmaceutical) was warmed to 85°C, and κ-carrageenan (0.4 parts by weight, GENUGEL JPE-126, from CP Kelco) was added thereto, and dissolved by sufficient agitation. Propylene glycol (3.6 parts by weight, from Wako Pure Chemical Industries, Ltd.) was added to the solution, and agitated at a temperature of 85°C. Thereafter, a 1.8-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 79 and 80)

Edible compositions were prepared respectively using the materials shown in Table 22 in the same manner as in Example 78. Instead of the κ-carrageenan, tamarind gum (Glyloid 3S, from DSP Gokyo Food & Chemical Co., Ltd.) was used in Example 79; and HM pectin (GENU PECTIN USP-H, from CP Kelco) was used in Example 80.

### (Comparative Example 45)

Purified water (8.0 parts by weight, Japanese pharmacopoeia purified water, from Kenei Pharmaceutical) was warmed to 85°C, and LM pectin (0.4 parts by weight, GENU PECTIN LM-102AS-J, from CP Kelco) was added thereto, and dissolved by sufficient agitation. Propylene glycol (3.6 parts by weight, from Wako Pure Chemical Industries, Ltd.) was added to the solution, and agitated at a temperature of 85°C. Thereafter, a 1.8-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Comparative Examples 46 to 49)

Edible compositions were prepared respectively using the materials shown in Table 22 in the same manner as in Comparative Example 45. Instead of the LM pectin, the following materials were used: Tara gum (MT120, from MRC Polysaccharide Co., Ltd.) in Comparative Example 46; locust bean gum (GENUGUM RL-200-J, from CP Kelco) in Comparative Example 47; xanthan gum (Rhaball gum GS-C, from DSP Gokyo Food & Chemical Co., Ltd.) in Comparative Example 48; and deacylated gellan gum (Kelcogel, from CP Kelco) in Comparative Example 49.

**[Table 22]**

| ingredient | Example [parts by weight] | | | | | | | | Comparative Example [parts by weight] | | | | |
|---|---|---|---|---|---|---|---|---|---|---|---|---|---|
| | 73 | 74 | 75 | 76 | 77 | 78 | 79 | 80 | 45 | 46 | 47 | 48 | 49 |
| Fish-derived water-soluble gelatin | 2.0 | - | - | - | - | - | - | - | - | - | - | - | - |
| Swine-derived low molecular weight gelatin | - | 2.0 | - | - | - | - | - | - | - | - | - | - | - |
| Acid-treated swine-derived getatin | - | - | 2.0 | - | - | - | - | - | - | - | - | - | - |
| Alkali-treated swine-delived gelatin | - | - | - | 2.0 | - | - | - | - | - | - | - | - | - |
| Alkali-treated bovine-origin gelatin | - | - | - | - | 2.0 | - | - | - | - | - | - | - | - |
| κ-Carrageenan | - | - | - | - | - | 0.4 | - | - | - | - | - | - | - |
| Tamarind gum | - | - | - | - | - | - | 0.4 | - | - | - | - | - | - |
| HM pectin | - | - | - | - | - | - | - | 0.4 | - | - | - | - | - |
| LM pectin | - | - | - | - | - | - | - | - | 0.4 | - | - | - | - |
| Tara gum | - | - | - | - | - | - | - | - | - | 0.4 | - | - | - |
| Locust bean gum | - | - | - | - | - | - | - | - | - | - | 0.4 | - | - |
| Xanthan gum | - | - | - | - | - | - | - | - | - | - | - | 0.4 | - |
| Deacylatad gellan gum | - | - | - | - | - | - | - | - | - | - | - | - | 0.4 |
| Purified water | (4.0) | (4.0) | (4.0) | (4.0) | (4.0) | (8.0) | (8.0) | 8.0) | (8.0) | (8.0) | (8.0) | (8.0) | (8.0) |
| Propylene glycol | 4.0 | 4.0 | 4.0 | 4.0 | 4.0 | 3.6 | 3.6 | 3.6 | 3.6 | 3.6 | 3.6 | 3.6 | 3.6 |
| Dispensed amount [g/blister] | 1.0 | 1.0 | 1.0 | 1.0 | 1.0 | 1.8 | 1.8 | 1.8 | 1.8 | 1.8 | 1.8 | 1.8 | 1.8 |

### (Example 81)

Purified water (4.0 parts by weight, Japanese pharmacopoeia purified water, from Kenei Pharmaceutical) was warmed to 40°C, and fish-derived water soluble gelatin (2.0 parts by weight, water-soluble gelatin CSF, from Nippi Inc.) was added thereto, and dissolved by sufficient agitation. Glycerin (4.0 parts by weight, from Wako Pure Chemical Industries, Ltd.) was added to the solution, and agitated at a temperature of 40°C. Thereafter, a 1.0-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Example 82)

Purified water (4.0 parts by weight, Japanese pharmacopoeia purified water, from Kenei Pharmaceutical) was warmed to 60°C, and swine-derived low molecular weight gelatin (2.0 parts by weight, swine bone gelatin AEP, from Nippi Inc.) was added thereto, and dissolved by sufficient agitation. Glycerin (4.0 parts by weight, from Wako Pure Chemical Industries, Ltd.) was added to the solution, and agitated at a temperature of 60°C. Thereafter, a 1.0-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 83 to 85)

Edible compositions were prepared respectively using the materials shown in Table 23 in the same manner as in Example 82. Instead of the swine-derived low molecular weight gelatin, the following materials were used: acid-treated swine-derived gelatin (AP-200F, from Nippi Inc.) in Example 83; alkali-treated swine-derived gelatin (BP-200F, from Nippi Inc.) in Example 84; and alkali-treated bovine-derived gelatin (AD4, from Nippi Inc.) in Example 85.

### (Example 86)

Purified water (8.0 parts by weight, Japanese pharmacopoeia purified water, from Kenei Pharmaceutical) was warmed to 85°C, and κ-carrageenan (0.4 parts by weight, GENUGEL JPE-126, from CP Kelco) was added thereto, and dissolved by sufficient agitation. Glycerin (3.6 parts by weight, from Wako Pure Chemical Industries, Ltd.) was added to the solution, and agitated at a temperature of 85°C. Thereafter, a 1.8-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 87 and 88)

Edible compositions were prepared respectively using the materials shown in Table 23 in the same manner as in Example 86. Instead of the κ-carrageenan, native-type gellan gum (Kelcogel LT100, from CP Kelco) was used in Example 87; and tamarind gum (Glyloid 3S, from DSP Gokyo Food & Chemical Co., Ltd.) was used in Example 88.

### (Comparative Example 50)

Purified water (8.0 parts by weight, Japanese pharmacopoeia purified water, from Kenei Pharmaceutical) was warmed to 85°C, and LM pectin (0.4 parts by weight, GENU PECTIN LM-102AS-J, from CP Kelco) was added thereto, and dissolved by sufficient agitation. Glycerin (3.6 parts by weight, from Wako Pure Chemical Industries, Ltd.) was added to the solution, and agitated at a temperature of 85°C. Thereafter, a 1.8-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Comparative Examples 51 to 54)

Edible compositions were prepared respectively using the materials shown in Table 23 in the same manner as in Comparative Example 50. Instead of the LM pectin, the following materials were used. Tara gum (MT120, from MRC Polysaccharide Co., Ltd.) in Comparative Example 51; locust bean gum (GENUGUM RL-200-J, from CP Kelco) in Comparative Example 52; xanthan gum (Rhaball gum GS-C, from DSP Gokyo Food & Chemical Co., Ltd.) in Comparative Example 53; and deacylated gellan gum (Kelcogel, from CP Kelco) in Comparative Example 54.

**[Table 23]**

| Ingredient | Example [parts by weight] | | | | | | | | Comparative Example [parts by weight] | | | | |
|---|---|---|---|---|---|---|---|---|---|---|---|---|---|
| | 81 | 82 | 83 | 84 | 85 | 86 | 87 | 88 | 50 | 51 | 52 | 53 | 54 |
| Fish-derived water-soluble gelatin | 2.0 | - | - | - | - | - | - | - | - | - | - | - | - |
| Swine-derived low molecular weight gelatin | - | 2.0 | - | - | - | - | - | - | - | - | - | - | - |
| Acid-treated swine-derived gelatin | - | - | 2.0 | - | - | - | - | - | - | - | - | - | - |
| Alkali-treated swine-derived gelatin | - | - | - | 2.0 | - | - | - | - | - | - | - | - | - |
| Alkali-treated bovine-origin gelatin | - | - | - | - | 2.0 | - | - | - | - | - | - | - | - |
| κ-carrageenan | - | - | - | - | - | 0.4 | - | - | - | - | - | - | - |
| Native-type gellan gum | - | - | - | - | - | - | 0.4 | - | - | - | - | - | - |
| Tamarind gum | - | - | - | - | - | - | - | 0.4 | - | - | - | - | - |
| LM pectin | - | - | - | - | - | - | - | - | 0.4 | - | - | - | - |
| Tara gum | - | - | - | - | - | - | - | - | - | 0.4 | - | - | - |
| Locust bean gum | - | | - | - | - | - | - | - | - | - | 0.4 | - | - |
| Xanthan gum | - | - | - | - | - | - | - | - | - | - | - | 0.4 | - |
| Deacylated gellan gum | - | - | - | - | - | - | - | - | - | - | - | - | 0.4 |
| Purified water | (4.0) | (4.0) | (4.0) | (4.0) | (4.0) | (8.0) | (8.0) | (8.0) | (8.0) | (8.0) | (8.0) | (8.0) | (8.0) |
| Glycerin | 4.0 | 4.0 | 4.0 | 4.0 | 4.0 | 3.6 | 3.6 | 3.6 | 3.6 | 3.6 | 3.6 | 3.6 | 3.6 |
| Dispensed amount [g/bilster] | 1.0 | 1.0 | 1.0 | 1.0 | 1.0 | 1.8 | 1.8 | 1.8 | 1.8 | 1.8 | 1.8 | 1.8 | 1.8 |

### (Example 89)

To purified water (1000 parts by weight, Japanese pharmacopoeia purified water, from Kenei Pharmaceutical) was added water soluble fish-derived gelatin (5.0 parts by weight, water-soluble gelatin CSF, from Nippi Inc.), and dissolved while heating at a temperature of 40°C, followed by standing to cool at a room temperature. The resulting solution was frozen with liquid nitrogen, and freeze-dried in a freeze dryer (freeze dryer DC400, from Yamato Scientific Co., Ltd.) overnight. The freeze-dried product (FD product) of the water-soluble fish-derived gelatin (2.0 parts by weight) was sufficiently agitated and then added to propylene glycol (6.0 parts by weight, from Wako Pure Chemical Industries, Ltd.), and dissolved while agitation at a temperature of 60°C. After the dissolution, a 0.6-g portion of the solution was dispensed to a plastic blister case (Cryomold standard (circle shape), from Sakura Finetek Japan Co., Ltd.), and allowed to cool at a room temperature. In this manner, an edible composition was provided.

### (Example 90)

To purified water (1000 parts by weight, Japanese pharmacopoeia purified water, from Kenei Pharmaceutical) was added swine-derived low molecular weight gelatin (5.0 parts by weight, swine bone gelatin AEP, from Nippi Inc.), and dissolved while heating at a temperature of 60°C, followed by standing to cool at a room temperature. The resulting solution was frozen with liquid nitrogen, and freeze-dried in a freeze dryer (freeze dryer DC400, from Yamato Scientific Co., Ltd.) overnight. The freeze-dried product (FD product) of the swine-derived low molecular weight gelatin (2.0 parts by weight) was sufficiently agitated and then added to propylene glycol (6.0 parts by weight, from Wako Pure Chemical Industries, Ltd.), and dissolved while agitation at a temperature of 60°C. After the dissolution, a 0.6-g portion of the solution was dispensed to a plastic blister case (Cryomold standard (circle shape), from Sakura Finetek Japan Co., Ltd.), and allowed to cool at a room temperature. In this manner, an edible composition was provided.

### (Examples 91 to 93)

Edible compositions were prepared respectively using the materials shown in Table 24 in the same manner as in Example 90. Instead of the swine-derived low molecular weight gelatin, the following materials were used: acid-treated swine-derived gelatin (AP-200F, from Nippi Inc.) in Example 91; alkali-treated swine-derived gelatin (BP-200F, from Nippi Inc.) in Example 92; and alkali-treated bovine-derived gelatin (AD4, from Nippi Inc.) in Example 93.

**[Table 24]**

| Ingredient | Example [parts by weight] | | | | |
|---|---|---|---|---|---|
| | 89 | 90 | 91 | 92 | 93 |
| Fish-derived water-soluble gelatin (FD product) | 2.0 | - | - | - | - |
| Swine-derived low molecular weight gelatin (FD product) | - | 2.0 | - | - | - |
| Acid-treated swine-derived gelatin (FD product) | - | - | 2.0 | - | - |
| Alkali-treated swine-derived gelatin (FD product) | - | - | - | 2.0 | - |
| Alkali-treated bovine-origin gelatin (FD product) | - | - | - | - | 2.0 |
| Propylene glycol | 6.0 | 6.0 | 6.0 | 6.0 | 6.0 |
| Dispensed amount [g/blister] | 0.6 | 0.6 | 0.6 | 0.6 | 0.6 |

### (Example 94)

To purified water (1000 parts by weight, Japanese pharmacopoeia purified water, from Kenei Pharmaceutical) was added fish-derived water soluble gelatin (5.0 parts by weight, water-soluble gelatin CSF, from Nippi Inc.), and dissolved while heating at a temperature of 40°C, followed by standing to cool at a room temperature. The resulting solution was frozen with liquid nitrogen, and freeze-dried in a freeze dryer (freeze dryer DC400, from Yamato Scientific Co., Ltd.) overnight. The freeze-dried product (FD product) of the water-soluble fish-derived gelatin (2.0 parts by weight) was sufficiently agitated and then added to glycerin (4.0 parts by weight, from Wako Pure Chemical Industries, Ltd.), and dissolved while agitation at a temperature of 60°C. After the dissolution, a 0.6-g portion of the solution was dispensed to a plastic blister case (Cryomold standard (circle shape), from Sakura Finetek Japan Co., Ltd.), and allowed to cool at a room temperature. In this manner, an edible composition was provided.

### (Example 95)

To purified water (1000 parts by weight, Japanese pharmacopoeia purified water, from Kenei Pharmaceutical) was added swine-derived low molecular weight gelatin (5.0 parts by weight, swine bone gelatin AEP, from Nippi Inc.), and dissolved while heating at a temperature of 60°C, followed by standing to cool at a room temperature. The resulting solution was frozen with liquid nitrogen, and freeze-dried in a freeze dryer (freeze dryer DC400, from Yamato Scientific Co., Ltd.) overnight. The freeze-dried product (FD product) of the swine-derived low molecular weight gelatin (2.0 parts by weight) was sufficiently agitated and then added to glycerin (4.0 parts by weight, from Wako Pure Chemical Industries, Ltd.), and dissolved while agitation at a temperature of 60°C. After the dissolution, a 0.6-g portion of the solution was dispensed to a plastic blister case (Cryomold standard (circle shape), from Sakura Finetek Japan Co., Ltd.), and allowed to cool at a room temperature. In this manner, an edible composition was provided.

### (Examples 96 to 98)

Edible compositions were prepared respectively using the materials shown in Table 25 in the same manner as in Example 95. Instead of the swine-derived low molecular weight gelatin, the following materials were used: acid-treated swine-derived gelatin (AP-200F, from Nippi Inc.) in Example 96; alkali-treated swine-derived gelatin (BP-200F, from Nippi Inc.) in Example 97; and alkali-treated bovine-derived gelatin (AD4, from Nippi Inc.) in Example 98.

### (Example 99)

To purified water (1000 parts by weight, Japanese pharmacopoeia purified water, from Kenei Pharmaceutical) was added κ-carrageenan (5.0 parts by weight, GENUGEL JPE-126, from CP Kelco), and dissolved while heating at a temperature of 70°C, followed by standing to cool at a room temperature. The resulting solution was frozen with liquid nitrogen, and freeze-dried in a freeze dryer (freeze dryer DC400, from Yamato Scientific Co., Ltd.) overnight. The freeze-dried product (FD product) of the κ-carrageenan (0.2 parts by weight) was sufficiently agitated and then added to glycerin (5.8 parts by weight, from Wako Pure Chemical Industries, Ltd.), and dissolved while agitation at a temperature of 85°C. After the dissolution, a 0.6-g portion of the solution was dispensed to a plastic blister case (Cryomold standard (circle shape), from Sakura Finetek Japan Co., Ltd.), and allowed to cool at a room temperature. In this manner, an edible composition was provided.

### (Examples 100 and 101)

Edible compositions were prepared respectively using the materials shown in Table 25 in the same manner as in Example 99. Instead of the κ-carrageenan, native-type gellan gum (Kelcogel LT100, from CP Kelco) was used in Example 100, and tamarind gum (Glyloid 3S, from DSP Gokyo Food & Chemical Co., Ltd.) was used in Example 101.

### (Comparative Example 55)

To purified water (1000 parts by weight, Japanese pharmacopoeia purified water, from Kenei Pharmaceutical) was added deacylated gellan gum (5.0 parts by weight, Kelcogel, from CP Kelco), and dissolved while heating at a temperature of 70°C, followed by standing to cool at a room temperature. The resulting solution was frozen with liquid nitrogen, and freeze-dried in a freeze dryer (freeze dryer DC400, from Yamato Scientific Co., Ltd.) overnight. The freeze-dried product (FD product) of the deacylated gellan gum (0.2 parts by weight) was sufficiently agitated and then added to glycerin (5.8 parts by weight, from Wako Pure Chemical Industries, Ltd.), and dissolved while agitation at a temperature of 85°C. After the dissolution, a 0.6-g portion of the solution was dispensed to a plastic blister case (Cryomold standard (circle shape), from Sakura Finetek Japan Co., Ltd.), and allowed to cool at a room temperature. In this manner, an edible composition was provided.

### (Comparative Example 56)

An edible composition was prepared using the materials shown in Table 25 in the same manner as in Comparative Example 55. LM pectin (GENU PECTIN LM-102AS-J, from CP Kelco) was used instead of the deacylated gellan gum.

**[Table 25]**

| Ingredient | Example [parts by weight] | | | | | | | | Comparative Example [parts by weight] | |
|---|---|---|---|---|---|---|---|---|---|---|
| | 94 | 95 | 96 | 97 | 98 | 99 | 100 | 101 | 55 | 56 |
| Fish-derived water-soluble gelatin (FD product) | 2.0 | - | - | - | - | - | - | - | - | - |
| Swine-derived low molecular weight gelatin (FD product) | - | 2.0 | - | - | - | - | - | - | - | - |
| Acid-treated swine-derived gelatin (FD product) | - | - | 2.0 | - | - | - | - | - | - | - |
| Alkali-treated swine-derived gelatin (FD product) | - | - | - | 2.0 | 2.0 | - | - | - | - | - |
| Alkali-treated bovine-origin gelatin (FD product) | - | - | - | - | 2.0 | - | - | - | - | - |
| κ-Cartageenan (FD product) | - | - | - | - | - | 0.2 | - | - | - | - |
| Native-type gellan gum (FD product) | - | - | - | - | - | - | 0.2 | - | - | - |
| Tamarind gum (FD product) | - | - | - | - | - | - | - | 0.2 | - | - |
| Deacylated gellan gum (FD product) | - | - | - | - | - | - | - | - | 0.2 | - |
| LM pectin (FD product) | - | - | - | - | - | - | - | - | - | 0.2 |
| Glycerin | 4.0 | 4.0 | 4.0 | 4.0 | 4.0 | 5.8 | 5.8 | 5.8 | 5.8 | 5.8 |
| Dispensed amount [g/blister] | 0.6 | 0.6 | 0.6 | 0.6 | 0.6 | 0.6 | 0.6 | 0.6 | 0.6 | 0.6 |

### (Example 102)

Purified water (9 parts by weight, Japanese pharmacopoeia purified water, from Kenei Pharmaceutical) was warmed to 60°C. Swine-derived low molecular weight gelatin (1.9 parts by weight, swine bone gelatin AEP, from Nippi Inc.) and carboxymethyl cellulose sodium (0.1 parts by weight, Cellogen PR-S, from DAI-ICHI KOGYO SEIYAKU CO., LTD.) were added to the water, and dissolved by sufficient agitation. Glycerin (4.0 parts by weight, from Wako Pure Chemical Industries, Ltd.) was added to the solution, and agitated at a temperature of 60°C. Thereafter, a 1.5-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 103 to 108)

Edible compositions were prepared respectively using the materials shown in Table 26 in the same manner as in Example 102. Instead of the carboxymethyl cellulose sodium, the following materials were used: κ-carrageenan (from GENUGEL JPE-126, from CP Kelco) in Example 103; τ-carrageenan, (GENUVISCO J-JPE, from CP Kelco) in Example 104; LM pectin (GENU PECTIN LM-102AS-J, from CP Kelco) in Example 105; deacylated gellan gum (Kelcogel, from CP Kelco) in Example 106; sodium alginate (KIMICA ALGIN IL-2, from Kimica Corporation) in Example 107; and psyllium seed gum (PG200, from MRC Polysaccharide Co., Ltd.) in Example 108.

**[Table 26]**

| Ingredient | Example [parts by weight] | | | | | | |
|---|---|---|---|---|---|---|---|
| | 102 | 103 | 104 | 105 | 106 | 107 | 108 |
| Swine-derived low molecular weight gelatin | 1.9 | 1.9 | 1.9 | 1.9 | 1.9 | 1.9 | 1.9 |
| Carboxymethyl cellulose sodium | 0.1 | - | - | - | - | - | - |
| κ-Carrageenan | - | 0.1 | - | - | - | - | - |
| ℩-Carrageenan | - | - | 0.1 | - | - | - | - |
| LM pectin | - | - | - | 0.1 | - | - | - |
| Deacylated gellan gum | - | - | - | - | 0.1 | - | - |
| Sodium alginate | - | - | - | - | - | 0.1 | - |
| Psyllium seed gum | - | - | - | - | - | - | 0.1 |
| Glycerin | 4.0 | 4.0 | 4.0 | 4.0 | 4.0 | 4.0 | 4.0 |
| Purified water | (9) | (9) | (9) | (9) | (9) | (9) | (9) |
| Dispensed amount [g/blister] | 1.5 | 1.5 | 1.5 | 1.5 | 1.5 | 1.5 | 1.5 |

### (Example 109)

Purified water (8.8 parts by weight, Japanese pharmacopoeia purified water, from Kenei Pharmaceutical) was warmed to 70°C. To the water was added κ-carrageenan (0.1 parts by weight, GENUGEL JPE-126, from CP Kelco) and xanthan gum (0.1 parts by weight, Rhaball gum GS-C, from DSP Gokyo Food & Chemical Co., Ltd.), and dissolved by sufficient agitation. Glycerin (4.0 parts by weight, from Wako Pure Chemical Industries, Ltd.) was added to the solution, and agitated at a temperature of 70°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 110 to 114)

Edible compositions were prepared respectively using the materials shown in Table 27 in the same manner as in Example 109. Instead of the xanthan gum, the following materials were used: τ-carrageenan, (GENUVISCO PJ-JPE, from CP Kelco) in Example 110; native-type gellan gum (Kelcogel LT100, from CP Kelco) in Example 111; psyllium seed gum (PG200, from MRC Polysaccharide Co., Ltd.) in Example 112; powdered tragacanth (powdered tragacanth, from Suzu Funmatsu Yakuhin K.K.) in Example 113; and sodium alginate (KIMICA ALGIN IL-2, from Kimica Corporation) in Example 114.

**[Table 27]**

| Ingredient | Example [parts by weight] | | | | | |
|---|---|---|---|---|---|---|
| | 109 | 110 | 111 | 112 | 113 | 114 |
| κ-Carrageenan | 0.1 | 0.1 | 0.1 | 0.1 | 0.1 | 0.1 |
| Xanthan gum | 0.1 | - | - | - | - | - |
| ℩-Carrageenan | - | 0.1 | - | - | - | - |
| Native-type gellan gum | - | - | 0.1 | - | - | - |
| Psyllium seed gum | - | - | - | 0.1 | - | - |
| Powdered tragacanth | - | - | - | - | 0.1 | - |
| Sodium alginate | - | - | - | - | - | 0.1 |
| Glycerin | 4.0 | 4.0 | 4.0 | 4.0 | 4.0 | 4.0 |
| Purified water | (8.8) | (8.8) | (8.8) | (8.8) | (8.8) | (8.8) |
| Dispensed amount [g/blister] | 1.9 | 1.9 | 1.9 | 1.9 | 1.9 | 1.9 |

### (Example 115)

Purified water (8.8 parts by weight, Japanese pharmacopoeia purified water, from Kenei Pharmaceutical) was warmed to 70°C. To the water was added τ-carrageenan, (0.1 parts by weight, GENUVISCO PJ-JPE, from CP Kelco) and carboxymethyl cellulose sodium (0.1 parts by weight, Cellogen PR-S, from DAI-ICHI KOGYO SEIYAKU CO., LTD.), and dissolved by sufficient agitation. Glycerin (4.0 parts by weight, from Wako Pure Chemical Industries, Ltd.) was added to the solution, and agitated at a temperature of 70°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 116 to 122)

Edible compositions were prepared respectively using the materials shown in Table 28 in the same manner as in Example 115. Instead of the carboxymethyl cellulose sodium, the following materials were used: sodium alginate (KIMICA ALGIN IL-2, from Kimica Corporation) in Example 116; xanthan gum (Rhaball gum GS-C, from DSP Gokyo Food & Chemical Co., Ltd.) in Example 117; λ-carrageenan (GENEVISCO CSM-2, from CP Kelco) in Comparative Example 118; deacylated gellan gum (Kelcogel, from CP Kelco) in Example 119; native-type gellan gum (Kelcogel LT100, from CP Kelco) in Example 120; psyllium seed gum (PG200, from MRC Polysaccharide Co., Ltd.) in Example 121; and powdered tragacanth (powdered tragacanth, from Suzu Funmatsu Yakuhin K.K.) in Example 122.

**[Table 28]**

| Ingredient | Example [parts by weight] | | | | | | | |
|---|---|---|---|---|---|---|---|---|
| | 115 | 116 | 117 | 118 | 119 | 120 | 121 | 122 |
| ℩-Carrageenan | 0.1 | 0.1 | 0.1 | 0.1 | 0.1 | 0.1 | 0.1 | 0.1 |
| Carboxymethyl cellulose sodium | 0.1 | - | - | - | - | - | - | - |
| Sodium alginate | - | 0.1 | - | - | - | - | - | - |
| Xanthan gum | - | - | 0.1 | - | - | - | - | - |
| λ-Carrageenan | - | - | - | 0.1 | - | - | - | - |
| Deacylated gellan gum | - | - | - | - | 0.1 | - | - | - |
| Native-type gellan gum | - | - | - | - | - | 0.1 | - | - |
| Psyllium seed gum | - | - | - | - | - | - | 0.1 | - |
| Powdered tragacanth | - | - | - | - | - | - | - | 0.1 |
| Glycin | 4.0 | 4.0 | 4.0 | 4.0 | 4.0 | 4.0 | 4.0 | 4.0 |
| Purfied water | (8.8) | (8.8) | (8.8) | (8.8) | (8.8) | (8.8) | (8.8) | (8.8) |
| Dispensed amount [g/blister] | 1.9 | 1.9 | 1.9 | 1.9 | 1.9 | 1.9 | 1.9 | 1.9 |

### (Example 123)

Purified water (8.8 parts by weight, Japanese pharmacopoeia purified water, from Kenei Pharmaceutical) was warmed to 70°C. To the water was added tamarind gum (0.1 parts by weight, Glyloid 3S, from DSP Gokyo Food & Chemical Co., Ltd.) and carboxymethyl cellulose sodium (0.1 parts by weight, Cellogen PR-S, from DAI-ICHI KOGYO SEIYAKU CO., LTD.), and dissolved by sufficient agitation. Glycerin (4.0 parts by weight, from Wako Pure Chemical Industries, Ltd.) was added to the solution, and agitated at a temperature of 70°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 124 to 134)

Edible compositions were prepared respectively using the materials shown in Table 29 in the same manner as in Example 123. Instead of the carboxymethyl cellulose sodium, the following materials were used: LM pectin (GENU PECTIN LM-102AS-J, from Sansyo,., Co., Ltd.) in Example 125; HM Pectin (GENU PECTIN USP-H, from Sansyo,., Co., Ltd.) in Example 126; sodium alginate (KIMICA ALGIN IL-2, from Kimica Corporation) in Example 127; xanthan gum (Rhaball gum GS-C, from DSP Gokyo Food & Chemical Co., Ltd.) in Example 128; 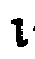 -carrageenan, (GENUVISCO PJ-JPE, from CP Kelco) in Example 129; λ-carrageenan (GENEVISCO CSM-2, from CP Kelco) in Example 130; deacylated gellan gum (Kelcogel, from CP Kelco) in Example 131; native-type gellan gum (Kelcogel LT100, from CP Kelco) in Example 132; psyllium seed gum (PG200, from MRC Polysaccharide Co., Ltd.) in Example 133; and powdered tragacanth (powdered tragacanth, from Suzu Funmatsu Yakuhin K.K.) in Example 134.

**[Table 29]**

| Ingredient | Example [parts by weight] | | | | | | | | | | | |
|---|---|---|---|---|---|---|---|---|---|---|---|---|
| | 123 | 124 | 125 | 126 | 127 | 128 | 129 | 130 | 131 | 132 | 133 | 134 |
| Tamarind gum | 0.1 | 0.1 | 0.1 | 0.1 | 0.1 | 0.1 | 0.1 | 0.1 | 0.1 | 0.1 | 0.1 | 0.1 |
| Carboxymethyl cellulose sodium | 0.1 | - | - | - | - | - | - | - | - | - | - | - |
| Carboxyvinyl polymer | - | 0.1 | - | - | - | - | - | - | - | - | - | - |
| LM pectin | - | - | 0.1 | - | - | - | - | - | - | - | - | - |
| HM pectin | - | - | - | 0.1 | - | - | - | - | - | - | - | - |
| Sodium alginate | - | - | - | - | 0.1 | - | - | - | - | - | - | - |
| Xanthan gum | - | - | - | - | - | 0.1 | - | - | - | - | - | - |
| ℩-Carrageenan | - | - | - | - | - | - | 0.1 | - | - | - | - | - |
| λ-Carrageenan | - | - | - | - | - | - | - | 0.1 | - | - | - | - |
| Deacylated gellan gum | - | - | - | - | - | - | - | - | 0.1 | - | - | - |
| Native-type gellan gum | - | - | - | - | - | - | - | - | - | 0.1 | - | - |
| Psyllium seed gum | - | - | - | - | - | - | - | - | - | - | 0.1 | - |
| Powdered tragacanth | - | - | - | - | - | - | - | - | - | - | - | 0.1 |
| Glycerin | 4.0 | 4.0 | 4.0 | 4.0 | 4.0 | 4.0 | 4.0 | 4.0 | 4.0 | 4.0 | 4.0 | 4.0 |
| Purified water | (8.8) | (8.8) | (8.8) | (8.8) | (8.8) | (8.8) | (8.8) | (8.8) | (8.8) | (8.8) | (8.8) | (8.8) |
| Dispensed amount [g/blister] | 1.9 | 1.9 | 1.9 | 1.9 | 1.9 | 1.9 | 1.9 | 1.9 | 1.9 | 1.9 | 1.9 | 1.9 |

### (Example 135)

Purified water (8.8 parts by weight, Japanese pharmacopoeia purified water, from Kenei Pharmaceutical) was warmed to 80°C. To the water was added xanthan gum (0.1 parts by weight, Rhaball gum GS-C, from DSP Gokyo Food & Chemical Co., Ltd.) and deacylated gellan gum (0.1 parts by weight, Kelcogel, from CP Kelco), and dissolved by sufficient agitation. Glycerin (4.0 parts by weight, from Wako Pure Chemical Industries, Ltd.) was added to the solution, and agitated at a temperature of 80°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Example 136)

An edible composition was prepared using the materials shown in Table 30 in the same manner as in Example 135. In Example 136, psyllium seed gum (PG200, from MRC Polysaccharide Co., Ltd.) was used instead of the deacylated gellan gum.

**[Table 30]**

| Ingredient | Example [parts by weight] | |
|---|---|---|
| | 135 | 136 |
| Xanthan gum | 0.1 | 0.1 |
| Deacylated gellan gum | 0.1 | - |
| Psyllium seed gum | - | 0.1 |
| Glycerin | 4.0 | 4.0 |
| Purified water | (8.8) | (8.8) |
| Dispensed amount [g/blister] | 1.9 | 1.9 |

### (Example 137)

Purified water (8.8 parts by weight, Japanese pharmacopoeia purified water, from Kenei Pharmaceutical) was warmed to 80°C. To the water was added deacylated gellan gum (0.1 parts by weight, Kelcogel, from CP Kelco) and carboxyvinyl polymer (0.1 parts by weight, Carbopol 971PNF), and dissolved by sufficient agitation. Glycerin (4.0 parts by weight, from Wako Pure Chemical Industries, Ltd.) was added to the solution, and agitated at a temperature of 80°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 138 to 144)

Edible compositions were prepared respectively using the materials shown in Table 31 in the same manner as in Example 137. Instead of the carboxyvinyl polymer, the following materials were used: LM pectin (GENU PECTIN LM-102AS-J, from Sansyo,., Co., Ltd.) in Example 138; HM pectin (GENU PECTIN USP-H, from Sansyo,., Co., Ltd.) in Example 139; sodium alginate (KIMICA ALGIN IL-2, from Kimica Corporation) in Example 140; λ-carrageenan, (GENEVISCO CSM-2, from CP Kelco) in Example 141; native-type gellan gum (Kelcogel LT100, from CP Kelco) in Example 142; psyllium seed gum (PG200, from MRC Polysaccharide Co., Ltd.) in Example 143; and powdered tragacanth (powdered tragacanth, from Suzu Funmatsu Yakuhin K.K.) in Example 144.

**[Table 31]**

| Ingredient | Example [parts by weight] | | | | | | | |
|---|---|---|---|---|---|---|---|---|
| | 137 | 138 | 139 | 140 | 141 | 142 | 143 | 144 |
| Deacylated gellan gum | 0.1 | 0.1 | 0.1 | 0.1 | 0.1 | 0.1 | 0.1 | 0.1 |
| Carboxyvinyl polymer | 0.1 | - | - | - | - | - | - | - |
| LM pectin | - | 0.1 | - | - | - | - | - | - |
| HM pectin | - | - | 0.1 | - | - | - | - | - |
| Sodium alginate | - | - | - | 0.1 | - | - | - | - |
| λ-carrageenan | - | - | - | - | 0.1 | - | - | - |
| Native-type gellan gum | - | - | - | - | - | 0.1 | - | - |
| Psyllium seed gum | - | - | - | - | - | - | 0.1 | - |
| Powdered tragacanth | - | - | - | - | - | - | - | 0.1 |
| Glycerin | 4.0 | 4.0 | 4.0 | 4.0 | 4.0 | 4.0 | 4.0 | 4.0 |
| Purified water | (8.8) | (8.8) | (8.8) | (8.8) | (8.8) | (8.8) | (8.8) | (8.8) |
| Dispensed amount [g/blister] | 1.9 | 1.9 | 1.9 | 1.9 | 1.9 | 1.9 | 1.9 | 1.9 |

### (Example 145)

Purified water (8.8 parts by weight, Japanese pharmacopoeia purified water, from Kenei Pharmaceutical) was warmed to 70°C. To the water was added LM pectin (0.1 parts by weight, GENU PECTIN LM-102AS-J, from Sansyo,., Co., Ltd.) and native-type gellan gum (0.1 parts by weight, Kelcogel LT100, from CP Kelco), and dissolved by sufficient agitation. Glycerin (4.0 parts by weight, from Wako Pure Chemical Industries, Ltd.) was added to the solution, and agitated at a temperature of 70°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 146 and 147)

Edible compositions were prepared respectively using the materials shown in Table 32 in the same manner as in Example 145. Instead of the native-type gellan gum, psyllium seed gum (PG200, from MRC Polysaccharide Co., Ltd.) was used in Example 146, and powdered tragacanth (powdered tragacanth, from Suzu Funmatsu Yakuhin K.K.) was used in Example 147.

**[Table 32]**

| Ingredient | Example [parts by weight] | | |
|---|---|---|---|
| | 145 | 146 | 147 |
| LM pectin | 0.1 | 0.1 | 0.1 |
| Native-type gellan gum | 0.1 | - | - |
| Psyllium seed gum | - | 0.1 | - |
| Powdered tragacanth | - | - | 0.1 |
| Glycerin | 4.0 | 4.0 | 4.0 |
| Purified water | (8.8) | (8.8) | (8.8) |
| Dispensed amount [g/blister] | 1.9 | 1.9 | 1.9 |

### (Example 148)

Purified water (8.8 parts by weight, Japanese pharmacopoeia purified water, from Kenei Pharmaceutical) was warmed to 70°C. To the water was added HM pectin (0.1 parts by weight, LM-102AS-J, from Sansyo,., Co., Ltd.) and carboxyvinyl polymer (0.1 parts by weight, Carbopol 971 PNF), and dissolved by sufficient agitation. Glycerin (4.0 parts by weight, from Wako Pure Chemical Industries, Ltd.) was added to the solution, and agitated at a temperature of 70°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Example 149)

An edible composition was prepared using the materials shown in Table 33 in the same manner as in Example 148. In Example 149, psyllium seed gum (PG200, from MRC Polysaccharide Co., Ltd.) was used instead of the carboxyvinyl polymer.

**[Table 33]**

| Ingredient | Example [parts by weight] | |
|---|---|---|
| | 148 | 149 |
| HM pectin | 0.1 | 0.1 |
| Carboxyvinyl polymer | 0.1 | - |
| Psyllium seed gum | - | 0.1 |
| Glycerin | 4.0 | 4.0 |
| Purified water | (8.8) | (8.8) |
| Dispensed amount [g/blister] | 1.9 | 1.9 |

### (Example 150)

Purified water (8.8 parts by weight, Japanese pharmacopoeia purified water, from Kenei Pharmaceutical) was warmed to 70°C. To the water was added native-type gellan gum (0.1 parts by weight, Kelcogel LT100, from CP Kelco) and carboxymethyl cellulose sodium (0.1 parts by weight, Cellogen PR-S, from DAI-ICHI KOGYO SEIYAKU CO., LTD.), and dissolved by sufficient agitation. Glycerin (4.0 parts by weight, from Wako Pure Chemical Industries, Ltd.) was added to the solution, and agitated at a temperature of 70°C. Thereafter, a 1.9-g portion of the mixture was dispensed to a plastic blister case (Cryomold standard (circle shape), from Sakura Finetek Japan Co., Ltd.), followed by drying in a vacuum dryer (DP63, from Yamato Scientific Co., Ltd.) at a temperature of 40°C for three hours so that water was evaporated. The residual water was further evaporated in a desiccator having a molecular sieve laid therein. In this manner, an edible composition was provided.

### (Examples 151 to 155)

Edible compositions were prepared respectively using the materials shown in Table 34 in the same manner as in Example 150. Instead of the carboxymethyl cellulose sodium, the following materials were used: carboxyvinyl polymer (CARBOPOL 971PNF) in Example 151; sodium alginate (KIMICA ALGIN IL-2, from Kimica Corporation) in Example 152; λ-carrageenan (GENEVISCO CSM-2, from CP Kelco) in Example 153; psyllium seed gum (PG200, from MRC Polysaccharide Co., Ltd.) in Example 154; and powdered tragacanth (powdered tragacanth, from Suzu Funmatsu Yakuhin K.K.) in Example 155.

**[Table 34]**

| Ingredient | Example [parts by weight] | | | | | |
|---|---|---|---|---|---|---|
| | 150 | 151 | 152 | 153 | 154 | 155 |
| Native-type gellan gum | 0.1 | 0.1 | 0.1 | 0.1 | 0.1 | 0.1 |
| Carboxymethyl cellulose sodium | 0.1 | - | - | - | - | - |
| Carboxyvinyl polymer | - | 0.1 | - | - | - | - |
| Sodium alginate | - | - | 0.1 | - | - | - |
| A-Carrageenan | - | - | - | 0.1 | - | - |
| Psyllium seed gum | - | - | - | - | 0.1 | - |
| Powdered tragacanth | - | - | - | - | - | 0.1 |
| Glycerin | 4.0 | 4.0 | 4.0 | 4.0 | 4.0 | 4.0 |
| Purified water | (8.8) | (8.8) | (8.8) | (8.8) | (8.8) | (8.8) |
| Dispensed amount [g/blister] | 1.9 | 1.9 | 1.9 | 1.9 | 1.9 | 1.9 |

### [Test method]

An examination was performed on whether each of the samples of the examples and the comparative examples returned to a jelly composition or jelly preparation by post-adding purified water, a 50% aqueous glycerol solution, and a drug-containing solution to the samples.
Whether the sample was re-jellified was evaluated as jelly recovery property. The time period required for the recovery to the jelly form was evaluated as jelly recovery time. The evaluation criteria are described below. Tables 35 to 42 show the results.
Meanwhile, the drug-containing solutions used were a cedar pollen extract solution (standardized therapeutic allergen extract, cedar pollen 2000 JAU/mL, from Torii Pharmaceutical Co., Ltd.), a house dust extract solution (therapeutic allergen extract for subcutaneous injection "Torii" house dust 1:10, from Torii Pharmaceutical Co., Ltd.), and a Ragweed pollen extract solution (therapeutic allergen extract for subcutaneous injection "Torii" Ragweed 1:10, from Torii Pharmaceutical Co., Ltd.).

### (1) Jelly recovery property (Evaluation 1, Evaluation 2, Evaluation 3)

Jelly recovery property was evaluated in the following cases: purified water was added (Evaluation 1) ; a 50 wt% aqueous glycerol solution was added (Evaluation 2); and a drug-containing solution was added (Evaluation 3). The evaluation criteria are as follows.
3: The sample was completely gelatinized to be a uniform jelly.
2: The sample was gelatinized to be a slightly-nonuniform jelly.
1: The sample was gelatinized to be a very soft and crumble jelly.
0: The sample was not gelatinized and became a viscous sol

### (2) Jelly recovery time (Evaluation 1, Evaluation 2, Evaluation 3)

Jelly recovery time was evaluated in the following cases: purified water was added (Evaluation 1) ; a 50 wt% aqueous glycerol solution was added (Evaluation 2); and a drug-containing solution was added (Evaluation 3). The evaluation criteria are as follows.
6: The sample became a formable jelly within 3 hours.
5: The sample became a formable jelly after between 3 to not more than 6 hours.
4: The sample became a formable jelly after between 6 to not more than 12 hours.
3: The sample became a formable jelly after between 12 to not more than 24 hours.
2: The sample became a formable jelly after between 24 to not more than 48 hours.
1: The sample became a formable jelly after between 48 to not more than 168 hours.
Meanwhile, the above test was not performed on the samples which were scored "0: The sample was not gelatinized and became a viscous sol" in the evaluation of the (1) Jelly recovery property, and those samples were scored "0".

**[Table 35]**

| Test No. | Sample | Amount of solution [mL] | Purified water added | | 50% aqueous glycerol solution added | | Total |
|---|---|---|---|---|---|---|---|
| | | | Evaluation 1 | | Evaluation 2 | | |
| | | | Jelly recovery property | Jelly recovery time | Jelly recovery property | Jelly recovery time | |
| 1 | Example 73 | 0.6 | 3 | 6 | 3 | 2 | 14 |
| 2 | Example 74 | 0.6 | 3 | 5 | 3 | 1 | 12 |
| 3 | Example 75 | 0.6 | 3 | 4 | 3 | 1 | 11 |
| 4 | Example 76 | 0.6 | 3 | 4 | 3 | 1 | 11 |
| 5 | Example 77 | 0.6 | 3 | 4 | 3 | 1 | 11 |
| 6 | Example 78 | 0.6 | 3 | 5 | 2 | 1 | 11 |
| 7 | Example 79 | 0.6 | 3 | 6 | 2 | 1 | 12 |
| 8 | Example 80 | 0.6 | 3 | 6 | 2 | 1 | 12 |
| 9 | Comparative Example 45 | 0.6 | 0 | 0 | 0 | 0 | 0 |
| 10 | Comparative Example 46 | 0.6 | 0 | 0 | 0 | 0 | 0 |
| 11 | Comparative Example 47 | 0.6 | 0 | 0 | 0 | 0 | 0 |
| 12 | Comparative Example 48 | 0.6 | 0 | 0 | 0 | 0 | 0 |
| 13 | Comparative Example 49 | 0.6 | 0 | 0 | 0 | 0 | 0 |

**[Table 36]**

| Test No. | Sample | Amount of solution [mL] | Purified water added | | 50% aqueous glycerol solution added | | Total |
|---|---|---|---|---|---|---|---|
| | | | Evaluation 1 | | Evaluation 2 | | |
| | | | Jelly recovery property | Jelly recovery time | Jelly recovery property | Jelly recovery time | |
| 14 | Example 81 | 0.6 | 3 | 6 | 3 | 2 | 14 |
| 15 | Example 82 | 0.6 | 3 | 5 | 3 | 1 | 12 |
| 16 | Example 83 | 0.6 | 3 | 5 | 3 | 1 | 12 |
| 17 | Example 84 | 0.6 | 3 | 5 | 3 | 1 | 12 |
| 18 | Example 85 | 0.6 | 3 | 5 | 3 | 1 | 12 |
| 19 | Example 86 | 0.6 | 3 | 5 | 2 | 1 | 11 |
| 20 | Example 87 | 0.6 | 3 | 5 | 2 | 1 | 11 |
| 21 | Example 88 | 0.6 | 3 | 6 | 3 | 1 | 13 |
| 22 | Comparative Example 50 | 0.6 | 0 | 0 | 0 | 0 | 0 |
| 23 | Comparative Example 51 | 0.6 | 0 | 0 | 0 | 0 | 0 |
| 24 | Comparative Example 52 | 0.6 | 0 | 0 | 0 | 0 | 0 |
| 25 | Comparative Example 53 | 0.6 | 0 | 0 | 0 | 0 | 0 |
| 26 | Comparative Example 54 | 0.6 | 0 | 0 | 0 | 0 | 0 |

**[Table 37]**

| Test No. | Sample | Amount of solution [mL] | Purified water added | | 50% aqueous glycerol solution added | | Total |
|---|---|---|---|---|---|---|---|
| | | | Evaluation 1 | | Evaluation 2 | | |
| | | | Jelly recovery property | Jelly recovery time | Jelly recovery property | Jelly recovery time | |
| 27 | Example 89 | 0.6 | 3 | 6 | 3 | 2 | 14 |
| 28 | Example 90 | 0.6 | 3 | 6 | 3 | 1 | 13 |
| 29 | Example 91 | 0.6 | 3 | 5 | 3 | 1 | 12 |
| 30 | Example 92 | 0.6 | 3 | 5 | 3 | 1 | 12 |
| 31 | Example 93 | 0.6 | 3 | 5 | 3 | 1 | 12 |
| 32 | Example 94 | 0.6 | 3 | 6 | 3 | 2 | 14 |
| 33 | Example 95 | 0.6 | 3 | 6 | 3 | 1 | 13 |
| 34 | Example 96 | 0.6 | 3 | 5 | 3 | 1 | 12 |
| 35 | Example 97 | 0.6 | 3 | 5 | 3 | 1 | 12 |
| 36 | Example 98 | 0.6 | 3 | 5 | 3 | 1 | 12 |
| 37 | Example 99 | 0.6 | 3 | 5 | 2 | 1 | 11 |
| 38 | Example 100 | 0.6 | 3 | 6 | 2 | 1 | 12 |
| 39 | Example 101 | 0.6 | 3 | 6 | 2 | 1 | 12 |
| 40 | Comparative Example 55 | 0.6 | 0 | 0 | 0 | 0 | 0 |
| 41 | Comparative Example 56 | 0.6 | 0 | 0 | 0 | 0 | 0 |

**[Table 38]**

| Test No. | Sample | Amount of solution [mL] | Purified water added | | 50% aqueous glycerol solution added | | Total |
|---|---|---|---|---|---|---|---|
| | | | Evaluation 1 | | Evaluation 2 | | |
| | | | Jelly recovery property | Jelly recovery time | Jelly recovery property | Jelly recovery time | |
| 42 | Example 73 | 0.1 | 3 | 6 | 3 | 4 | 16 |
| 43 | Example 73 | 1.0 | 3 | 6 | 3 | 1 | 13 |
| 44 | Example 73 | 3.0 | 3 | 6 | 2 | 1 | 12 |
| 45 | Example 73 | 8.0 | 2 | 4 | 1 | 1 | 8 |
| 46 | Example 73 | 10.0 | 1 | 3 | 1 | 1 | 6 |
| 47 | Example 82 | 0.1 | 3 | 6 | 3 | 3 | 15 |
| 48 | Example 82 | 1.0 | 3 | 6 | 2 | 1 | 12 |
| 49 | Example 82 | 3.0 | 3 | 5 | 1 | 1 | 10 |
| 50 | Example 82 | 8.0 | 3 | 4 | 1 | 1 | 9 |
| 51 | Example 82 | 10.0 | 1 | 3 | 1 | 1 | 6 |
| 52 | Example 85 | 0.1 | 3 | 6 | 3 | 3 | 15 |
| 53 | Example 85 | 1.0 | 3 | 4 | 1 | 1 | 9 |
| 54 | Example 85 | 3.0 | 3 | 2 | 1 | 1 | 7 |
| 55 | Example 85 | 8.0 | 2 | 1 | 1 | 1 | 5 |
| 56 | Example 85 | 10.0 | 2 | 1 | 1 | 1 | 5 |

**[Table 39]**

| Test No. | Sample | Drug-containing solution, Amount | Evaluation 3 | | |
|---|---|---|---|---|---|
| | | | Jelly recovery property | Jelly recovery time | Total |
| 57 | Example 82 | Cedar pollen extract solution 0.1mL | 3 | 4 | 7 |
| 58 | Example 82 | Cedar pollen extract solution 1mL | 3 | 1 | 4 |
| 59 | Example 82 | Cedar pollen extract solution 2mL | 3 | 1 | 4 |
| 60 | Example 82 | House dust extract solution 0.1mL | 3 | 6 | 9 |
| 61 | Example 82 | House dust extract solution 1mL | 3 | 6 | 9 |
| 62 | Example 82 | House dust extract solution 2mL | 3 | 4 | 7 |
| 63 | Example 85 | Ragweed pollen extract solution 0.1mL | 3 | 3 | 6 |
| 64 | Example 85 | Ragweed pollen extract solution 1mL | 3 | 3 | 6 |
| 65 | Example 85 | Ragweed pollen extract solution 2mL | 3 | 2 | 5 |
| 66 | Comparative Example 52 | Cedar pollen extract solution 0.1mL | 0 | 0 | 0 |
| 67 | (Comparative Example 52 | Cedar pollen extract solution 1mL | 0 | 0 | 0 |
| 68 | Comparative Example 52 | Cedar pollen extract solution 2mL | 0 | 0 | 0 |

Comparison between the results of Examples 73 to 101 and the results of Comparative Examples 45 to 56 in Tables 35 to 37 clearly revealed that the samples of those examples excellently absorbed purified water and rapidly became a jelly. It was also revealed that the samples of Examples 73 to 101 excellently absorbed a 50% aqueous glycerol solution; however, it took a considerable time before restoring to a jelly. Moreover, in the samples shown in Table 38 to which purified water and a 50% aqueous glycerol solution were added each in an amount of 0.1 to 10 mL, only the samples to which purified water was added in an amount of 0.1 to 3 mL and the samples to which a 50% aqueous glycerol solution was added in an amount of 0.1 mL fully became a jelly. Furthermore, as shown in Table 39, only the samples to which a house dust extract solution, which is a glycerin-free drug-containing solution, achieved good results. The samples to which was added a cedar pollen extract solution or an Ragweed pollen extract solution, which is a drug-containing solution including glycerin, required a considerable time before restoring to a jelly.

**[Table 40]**

| Test No. | Sample | Amount of solution [mL] | Purified water added | | 50% aqueous glycerol solution added | | Total |
|---|---|---|---|---|---|---|---|
| | | | Evaluation 1 | | Evaluation 2 | | |
| | | | Jelly recovery property | Jelly recovery time | Jelly recovery property | Jelly recovery time | |
| 69 | Example 102 | 0.6 | 3 | 6 | 3 | 4 | 16 |
| 70 | Example 103 | 0.6 | 3 | 6 | 3 | 2 | 14 |
| 71 | Example 104 | 0.6 | 3 | 6 | 3 | 4 | 16 |
| 72 | Example 105 | 0.6 | 3 | 6 | 3 | 4 | 16 |
| 73 | Example 106 | 0.6 | 3 | 6 | 3 | 3 | 15 |
| 74 | Example 107 | 0.6 | 3 | 6 | 3 | 3 | 15 |
| 75 | Example 108 | 0.6 | 3 | 6 | 3 | 3 | 15 |
| 76 | Example 109 | 0.6 | 3 | 6 | 3 | 1 | 13 |
| 77 | Example 110 | 0.6 | 3 | 6 | 3 | 3 | 15 |
| 78 | Example 111 | 0.6 | 3 | 6 | 2 | 2 | 13 |
| 79 | Example 112 | 0.6 | 3 | 6 | 3 | 3 | 15 |
| 80 | Example 113 | 0.6 | 3 | 6 | 2 | 3 | 14 |
| 81 | Example 114 | 0.6 | 3 | 6 | 2 | 2 | 13 |
| 82 | Example 115 | 0.6 | 3 | 6 | 2 | 5 | 16 |
| 83 | Example 116 | 0.6 | 3 | 6 | 3 | 4 | 16 |
| 84 | Example 117 | 0.6 | 3 | 6 | 3 | 2 | 14 |
| 85 | Example 118 | 0.6 | 3 | 6 | 3 | 5 | 17 |
| 86 | Example 119 | 0.6 | 3 | 6 | 3 | 4 | 16 |
| 87 | Example 120 | 0.6 | 3 | 6 | 3 | 3 | 15 |
| 88 | Example 121 | 0.6 | 3 | 6 | 3 | 4 | 16 |
| 89 | Example 122 | 0.6 | 3 | 6 | 3 | 3 | 15 |
| 90 | Example 123 | 0.6 | 3 | 6 | 3 | 5 | 17 |
| 91 | Example 124 | 0-6 | 3 | 6 | 3 | 6 | 18 |
| 92 | Example 125 | 0.6 | 3 | 6 | 3 | 4 | 16 |
| 93 | Example 126 | 0.6 | 3 | 6 | 3 | 4 | 16 |
| 94 | Example 127 | 0.6 | 3 | 6 | 3 | 4 | 16 |
| 95 | Example 128 | 0.6 | 3 | 6 | 3 | 3 | 15 |
| 96 | Example 129 | 0.6 | 3 | 6 | 3 | 5 | 17 |
| 97 | Example 130 | 0.6 | 3 | 6 | 3 | 5 | 17 |
| 98 | Example 131 | 0.6 | 3 | 6 | 3 | 5 | 17 |
| 99 | Example 132 | 0.6 | 3 | 6 | 3 | 4 | 16 |
| 100 | Example 133 | 0.6 | 3 | 6 | 3 | 5 | 17 |
| 101 | Example 134 | 0.6 | 3 | 6 | 3 | 4 | 16 |

**[Table 41]**

| Test No. | Sample | Amount of solution [mL] | Purified water added | | 50% aqueous glycerol solution added | | Total |
|---|---|---|---|---|---|---|---|
| | | | Evaluation 1 | | Evaluation 2 | | |
| | | | Jelly recovery property | Jelly recovery time | Jelly recovery property | Jelly recovery time | |
| 102 | Example 135 | 0.6 | 3 | 6 | 3 | 1 | 13 |
| 103 | Example 136 | 0.6 | 3 | 6 | 3 | 2 | 14 |
| 104 | Example 137 | 0.6 | 3 | 6 | 3 | 4 | 16 |
| 105 | Example 138 | 0.6 | 3 | 6 | 3 | 4 | 16 |
| 106 | Example 139 | 0.6 | 3 | 6 | 2 | 3 | 14 |
| 107 | Example 140 | 0.6 | 3 | 6 | 3 | 4 | 16 |
| 108 | Example 141 | 0.6 | 3 | 6 | 3 | 4 | 16 |
| 109 | Example 142 | 0.6 | 3 | 6 | 2 | 4 | 15 |
| 110 | Example 143 | 0.6 | 3 | 6 | 3 | 4 | 16 |
| 111 | Example 144 | 0.6 | 3 | 6 | 3 | 2 | 14 |
| 112 | Example 145 | 0.6 | 3 | 6 | 2 | 4 | 15 |
| 113 | Example 146 | 0.6 | 3 | 6 | 2 | 5 | 16 |
| 114 | Example 147 | 0.6 | 3 | 6 | 3 | 4 | 16 |
| 115 | Example 148 | 0.6 | 3 | 6 | 3 | 5 | 17 |
| 116 | Example 149 | 0.6 | 3 | 6 | 3 | 4 | 16 |
| 117 | Example 150 | 0.6 | 3 | 6 | 3 | 3 | 15 |
| 118 | Example 151 | 0.6 | 3 | 6 | 3 | 4 | 16 |
| 119 | Example 152 | 0.6 | 3 | 6 | 3 | 3 | 15 |
| 120 | Example 153 | 0.6 | 3 | 6 | 3 | 4 | 16 |
| 121 | Example 154 | 0.6 | 3 | 6 | 3 | 3 | 15 |
| 122 | Example 155 | 0.6 | 3 | 6 | 3 | 3 | 15 |

**[Table 42]**

| Test No. | Sample | Drug-containing solution, Amount | Evaluation 3 | | |
|---|---|---|---|---|---|
| | | | Jelly recovery property | Jelly recovery time | Total |
| 123 | Example 124 | Cedar pollen extract solution 0.1mL | 3 | 6 | 9 |
| 124 | Example 124 | Cedar pollen extract solution 1mL | 3 | 5 | 8 |
| 125 | Example 124 | Cedar pollen extract solution 2mL | 3 | 2 | 5 |
| 126 | Example 124 | House dust extract solution 0.1mL | 3 | 6 | 9 |
| 127 | Example 124 | House dust extract solution 1mL | 3 | 6 | 9 |
| 128 | Example 124 | House dust extract solution 2mL | 3 | 6 | 9 |
| 129 | Example 129 | Ragweed pollen extract solution extract solution 0.1mL | 3 | 6 | 9 |
| 130 | Example 129 | Ragweed pollen extract solution extract solution 1mL | 3 | 4 | 7 |
| 131 | Example 129 | Ragweed pollen extract solution extract solution 2mL | 3 | 2 | 5 |

As shown in Tables 40 to 41, the edible compositions of Examples 102 to 155 achieved good results in all the evaluation items. As shown in Table 42, the edible compositions of Examples 124 and 129 practically achieved sufficiently good results even in the case where they were mixed with a cedar pollen extract solution or an Ragweed pollen extract solution, which is a drug-containing solution including glycerin.

The edible jelly composition of the present invention is a jelly-like (as a result, easy-to-swallow), intraorally soluble edible jelly composition although it is a jelly-like composition preferably free of water.
Further, the dissolution time of the edible jelly composition of the present invention can be easily adjusted by adjusting the amount of the nonvolatile organic solvent. Since the dissolution time is adjustable, the composition of the present invention can be suitably formed into a dosage form of a pharmaceutical product to be absorbed through the oral mucosa and sublingual mucosa that requires intraoral residence time, and is also suitable for sublingual hyposensitization therapy in which the body is sensitized to allergens through the sublingual mucosa.
Since the edible jelly composition of the present invention is free of water, the composition does not need a sterilization step or addition of an antiseptic, which are required for common jelly. Thus, the composition of the present invention is advantageous in the production cost, and is suitable for forms of dietary supplements and pharmaceutical products for patients who need water restriction.
The edible jelly composition of the present invention suitably contains gelatin and polyols such as glycerin and propylene glycol which are commonly known to improve storage stability of proteins and peptides. Thus, the composition is expected to stably maintain, in particular, proteins and peptides.
The edible jelly composition of the present invention may naturally be swallowed as it is, or may be rapidly dissolved intraorally and then swallowed. In addition, the intraoral dissolution time is adjustable, and thus the composition can be expected to be absorbed through the oral mucosa and sublingual mucosa. Since the composition can be perfectly dissolved by body temperature and thus causes no feeling of residues, and since the composition is free of water, the jelly preparation comprising the edible jelly composition of the present invention can greatly improve the QOL of patients who need water restriction, patients with dysphagia, and caregivers.

## Claims

1. An edible jelly composition, comprising:
a gelling agent; and
a nonvolatile organic solvent compatible with the gelling agent.

2. The edible jelly composition according to claim 1, which is free of water.

3. The edible jelly composition according to claim 1 or 2,
wherein the nonvolatile organic solvent is a polyhydric alcohol having two to four OH groups and four or less carbon atoms per molecule.

4. The edible jelly composition according to any one of claims 1 to 3,
wherein the nonvolatile organic solvent is at least one selected from the group consisting of glycerin, glycerin derivative, propylene glycol, and propylene glycol derivatives.

5. The edible jelly composition according to any one of claims 1 to 4,
wherein the gelling agent is at least one of gelatin and a polysaccharide gelling agent.

6. The edible jelly composition according to claim 5,
wherein the gelatin has a random coil structure which is formed by transforming a helix structure of a slightly water soluble gelatin.

7. The edible jelly composition according to claim 5,
wherein the polysaccharide gelling agent is at least one selected from the group consisting of κ-carrageenan, xanthan gum, gellan gum, tamarind gum, and pectin.

8. The edible jelly composition according to any one of claims 1 to 4,
wherein the gelling agent contains at least one selected from the group consisting of gelatin, κ-carrageenan, xanthan gum, deacylated gellan gum, tamarind gum, LM pectin, and HM pectin, and
the nonvolatile organic solvent compatible with the gelling agent is at least one of propylene glycol and a propylene glycol derivative.

9. The edible jelly composition according to any one of claims 1 to 4,
wherein the gelling agent contains at least one selected from the group consisting of gelatin, κ-carrageenan, native gellan gum, deacylated gellan gum, tamarind gum, LM pectin, 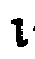 -carrageenan/carboxymethyl cellulose sodium, 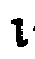 -carrageenan/sodium alginate, 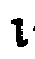 -carrageenan/xanthan gum, 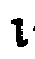 -carrageenan/λ-carrageenan, 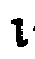 -carrageenan/deacylated gellan gum, 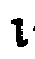 -carrageenan/psyllium seed gum, 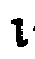 -carrageenan/powdered tragacanth, xanthan gum/deacylated gellan gum, xanthan gum/psyllium seed gum, deacylated gellan gum/carboxy vinyl polymer, deacylated gellan gum/LM pectin, deacylated gellan gum/sodium alginate, deacylated gellan gum/λ-carrageenan, deacylated gellan gum/psyllium seed gum, deacylated gellan gum/powdered tragacanth, LM pectin/psyllium seed gum, and LM pectin/powdered tragacanth, and
the nonvolatile organic solvent compatible with the gelling agent is at least one of glycerin and a glycerin derivative.

10. The edible jelly composition according to any one of claims 1 to 9,
wherein the nonvolatile organic solvent contains at least one of glycerin and propylene glycol, and further contains, as a solution-absorption enhancer, at least one selected from the group consisting of carboxymethyl cellulose sodium, LM pectin, sodium alginate, xanthan gum, 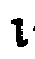 -carrageenan, λ-carrageenan, deacylated gellan gum, psyllium seed gum, and powdered tragacanth.

11. The edible jelly composition according to any one of claims 1 to 10,
wherein an amount of the gelling agent is 0.1 to 40% by weight of the whole amount of the composition.

12. The edible jelly composition according to any one of claims 1 to 11,
wherein an amount of the nonvolatile organic solvent is 10 to 99% by weight of the whole amount of the composition.

13. A jelly preparation, comprising:
the edible jelly composition according to any one of claims 1 to 12; and
a drug.

14. A jelly preparation, comprising:
the edible jelly composition according to any one of claims 1 to 12; and
a drug-containing solution.

15. The jelly preparation according to claim 14,
wherein the drug-containing solution is an injectable solution or an oral solution.

16. The jelly preparation according to claim 14 or 15,
wherein a solvent of the drug-containing solution is at least one selected from the group consisting of water, glycerin, and propylene glycol.

17. A method for producing a jelly preparation, the method comprising:
mixing a gelling agent, a nonvolatile organic solvent compatible with the gelling agent, and a drug to prepare a gelling agent solution;
dispensing or applying the gelling agent solution; and
standing the solution to cool or cooling the solution to solidify the solution.

18. A method for producing a jelly preparation, the method comprising:
mixing water, a gelling agent, and a nonvolatile organic solvent compatible with the gelling agent to form a mixture, and then heating the mixture to dissolve the gelling agent, to thereby prepare a nonvolatile organic solvent-containing gelling agent solution;
dispensing or applying the nonvolatile organic solvent-containing gelling agent solution;
standing the solution to cool or cooling the solution to solidify the solution, to thereby prepare an edible jelly composition; and
adding a drug-containing solution to the edible jelly composition to prepare a jelly preparation.

19. A method for producing a jelly preparation, the method comprising:
mixing water and a gelling agent to form a mixture, and then heating the mixture to dissolve the gelling agent, to thereby prepare a gelling agent solution;
freeze-drying or spray-drying the gelling agent solution to prepare a freely soluble gelling agent;
mixing the freely soluble gelling agent and a nonvolatile organic solvent compatible with the freely soluble gelling agent to form a mixture, and then heating the mixture to dissolve the freely soluble gelling agent, to thereby prepare a nonvolatile organic solvent-containing gelling agent solution;
dispensing or applying the nonvolatile organic solvent-containing gelling agent solution;
standing the solution to cool or cooling the solution to solidify the solution, to thereby prepare an edible jelly composition; and
adding a drug-containing solution to the edible jelly composition to prepare a jelly preparation.

20. A method for producing a jelly preparation, the method comprising:
mixing water and a gelling agent to form a mixture, and then heating the mixture to dissolve the gelling agent, to thereby prepare a gelling agent solution;
freeze-drying or spray-drying the gelling agent solution to prepare a freely soluble gelling agent;
mixing the freely soluble gelling agent, a drug, and a nonvolatile organic solvent compatible with the freely soluble gelling agent to prepare a nonvolatile organic solvent-containing gelling agent solution;
dispensing or applying the nonvolatile organic solvent-containing gelling agent solution; and
standing the solution to cool or cooling the solution to solidify the solution.
